# EUROPEAN PATENT APPLICATION

(11) **EP 3 498 270 A1**
(43) Date of publication of application: **19.06.2019**
(21) Application number: 18203123.7
(22) Date of filing: 29.10.2018
(51) Int. Cl.: A61K 31/17, A61K 31/407, A61K 31/519, A61K 31/704, A61K 31/7068, A61K 9/00, A61K 9/06, A61K 47/10, A61P 35/00

(54) **SLOW RELEASE FORMULATIONS OF CELL CYCLE REGULATORS AND ANTI-CANCER AGENTS FOR LOCAL TREATMENT OF SOLID CANCER**

(30) Priority: 30.10.2017 EP 17199125
(71) Applicant: Urogen Pharma Ltd., 4365007 Ra`anana (IL)
(72) Inventor: Strauss-Ayali, Dalit, 4493500 Sde Warburg (IL); Hakim, Gil, 4358414 Ra'anana (IL); Konorty, Marina, 4644650 Herzliya (IL); Friedman, Astar, 4974447 Petah Tikva (IL)
(74) Representative: Schulz Junghans Patentanwälte PartGmbB

(57) **Abstract**

The invention relates to the use of a cell cycle modulator capable of arresting the cell cycle in the S and/or G1 phase for the treatment of cancer of a body cavity. The cell cycle modulator comprises in a first composition comprising a first sustained release composition component, such as a thermoreversible hydrogel, and wherein said first composition is administered locally to the body cavity. Administration of the first composition is followed by administration of a second composition comprising at least one anti-cancer agent to the body cavity.

## Description

The invention relates to a pharmaceutical combination medicament for treating cancer, specifically urinary tract cancer, wherein the combination medicament comprises at least one cell cycle modulator, and at least one anti-cancer drug for local treatment, embedded in, and slowly released from, a biocompatible sustained polymer composition.

Urothelial carcinoma of the urinary bladder represents more than 90% of all bladder cancers, about 80% of which are non-muscle invasive bladder cancer (NMIBC), a form of superficial cancer. Transurethral resection and radical cystectomy remain the mainstay of treatment for NMIBC and MIBC (muscle invasive bladder cancer), respectively. Intravesical therapy of Bacillus calmette-Guerin (BCG) and mitomycin C (MMC) are preferable adjuvant therapies to surgery aiming at reducing disease recurrence and progression. The major drawback for intravesical instillation is the short exposure time of tissue to the drug due to urine creation and voiding that dilutes and displaces drug from the area of treatment.

It is well known that malignant neoplasms consist of heterogeneous cells which proliferate asynchronously. As a result, administered cytotoxic drugs will affect mainly those malignant cells that are at the specific cell cycle phase at which the drug is most efficacious. The rest of the cells will be affected only if the exposure time is long enough to permit the cells to cycle to the cell phase that is most sensitive to the activity of a specific chemotherapeutic drug. In the bladder and other body cavities of continual secretion and voiding, exposure time of a cancer target to cytotoxic drugs will be limited. Therefore, the treatment efficiency will be relatively low and will require repeating treatment sessions.

Thus, there remains a need for improvements in delivery and efficacy of non-surgical cancer treatments of cancers in secreting and voiding areas of the body such as the urinary tract, and particularly the bladder and the upper urinary tract.

The present invention therefore seeks to provide new combinations of cell cycle modulators and at least one anti-cancer agent for the treatment of proliferative disorders, especially cancer. More specifically, the invention relates to unexpected effects associated with increased efficacy for treating cancer while using cell cycle modifiers and at least one anti-cancer agent, each could be embedded in a sustained polymer matrix such as but not limited to a thermoreversible matrix, such as a thermoreversible hydrogel matrix, and locally administered to the affected body cavity, such as by minimally invasive means, by way of natural orifices or intravesical means.

### Summary

The inventors have surprisingly found that sustained local treatment of a body cavity cancer using a cell cycle modulator (CCM), followed by sustained local treatment with a chemotherapeutic agent can provide a superior cytotoxic, anti-cancer effect in comparison to treatment with either agent alone or simultaneously provided.

The fluid-filled environment of many body cavities presents a challenge to achieving such sustained treatments. Therefore, the treatments described herein are provided in sustained release compositions, such as hydrogel compositions, including but not limited to polymeric compositions having reverse thermal gelation (RTG) properties which enable a liquid mixture with a specific drug, which can be applied to an internal body cavity at a temperature below its gelation point. The composition will then solidify at body temperature or below (such compositions herein are also referred to as a "thermoreversible hydrogel"). The hydrogel composition is mixed with the specific active pharmaceutical agents to produce compositions for local sustained treatment.

Accordingly, provided herein are cell cycle modulators (CCMs) capable of arresting the cell cycle or synchronizing the cells in the G1, S, G2 or M phase for the treatment of cancer of a body cavity, wherein the cell cycle modulator is administered in a first composition that comprises optionally, a first sustained release composition component, for example a thermoreversible hydrogel composition, wherein the CCM and optionally the sustained release composition, for example a thermoreversible hydrogel composition, is administered locally to said body cavity, is followed by administration into said body cavity of a second composition that comprises at least one anti cancer agent and a optionally a second sustained release composition, for example a second thermoreversible hydrogel component.

Additionally described are combination medicaments that comprise:
1. A first composition comprising a cell cycle modulator capable of arresting the cell cycle or synchronizing the cells in the G1, S, G2 or M phase or any intermediate phase, and optionally a sustained release composition component, such as a thermoreversible hydrogel component.
2. At least one chemotherapeutic agent optionally combined with a sustained release composition, such as a thermoreversible hydrogel composition.

In particular embodiments, radiotherapy is provided prior to, or as an alternative to the second composition containing a chemotherapeutic agent.

Further described are packing units that include a first sustained release composition, for example a thermoreversible hydrogel composition, including a cell cycle modulator capable of arresting the cell cycle or synchronizing cells in the G1, S, G2 or M phase or any intermediate phase thereof, a second sustained release composition, for example a second thermoreversible hydrogel composition, including at least one chemotherapeutic agent, and instructions for preparation and use of the compositions in the packing unit.

Various embodiments of the provided compositions, medicaments, and packing units that include a variety of CCMs and chemotherapeutic agents, in a variety of sustained release compositions, including a variety of thermoreversible hydrogels, and in a variety of administration timing regimes, are also provided herein.

### Detailed Description

### I. Abbreviations

- BCG: Bacilus-Calmette-Guerin
- Gem: Gemcitabine
- Doxo: Doxorubicin
- CCM: Cell cycle modulator
- HU: Hydroxyurea
- HPMC: Hydroxyl propylmethyl cellulose
- MMC: Mitomycin C
- PEG: Polyethylene glycol
- PEO: Polyethylene oxide
- PVP: Polyvinylpyrrolidone
- PPO: Polypropylene oxide

### II. Terms

Unless otherwise noted, technical terms are used according to conventional usage. Definitions of common terms in molecular biology can be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

Unless otherwise explained, all technical and scientific terms used herein have the same meaning as commonly understood by one of ordinary skill in the art to which this disclosure belongs. The singular terms "a," "an," and "the" include plural referents unless context clearly indicates otherwise. Similarly, the word "or" is intended to include "and" unless the context clearly indicates otherwise. It is further to be understood that as applicable, all base sizes or amino acid sizes, and all molecular weight or molecular mass values, given for nucleic acids or polypeptides are approximate, and are provided for description. Although methods and materials similar or equivalent to those described herein can be used in the practice or testing of this disclosure, suitable methods and materials are described below. The term "comprises" means "includes." The abbreviation, "*e.g.*" is derived from the Latin *exempli gratia*, and is used herein to indicate a non-limiting example. Thus, the abbreviation "*e.g.*" is synonymous with the term "for example." In case of conflict, the present specification, including explanations of terms, will control. In addition, all the materials, methods, and examples are illustrative and not intended to be limiting.

**Administration:** The introduction of a composition into a subject by a chosen route. Administration of an active compound or composition can be by any route known to one of skill in the art, and as appropriate for the compound and the delivery system. For example, the compositions for use in the described methods are typically administered locally to the inside surface of a body cavity, such as by intravesical instillation or through a percutaneous route.

**Analog, derivative or mimetic:** An analog is a molecule that differs in chemical structure from a parent compound, for example a homolog (differing by an increment in the chemical structure, such as a difference in the length of an alkyl chain), a molecular fragment, a structure that differs by one or more functional groups, a change in ionization. Structural analogs are often found using quantitative structure activity relationships (QSAR), with techniques such as those disclosed in *Remington (*The Science and Practice of Pharmacology, 19th Edition (1995), chapter 28). A derivative is a biologically active molecule derived from the base structure. A mimetic is a molecule that mimics the activity of another molecule, such as a biologically active molecule. Biologically active molecules can include chemical structures that mimic the biological activities of a compound. It is acknowledged that these terms may overlap in some circumstances. In particular embodiments of the claimed methods, analogs, derivatives, or mimetics having comparable activity to the expressly recited compounds can be used in place of the recited compounds.

**Body cavity:** Any space either empty or fluid-filled that is located with (internal to) a multicellular organism. In particular embodiments, a body cavity can include other body cavities. For example, the mammalian pelvic cavity includes the bladder, and the thoracic cavity includes the upper gastro-intestinal tract and cavities such as the esophagus. In particular embodiments, a body cavity can be the urinary tract or any component thereof, such as one or both kidneys, renal pelvis, calyces, one or both ureters, the bladder, or the urethra.

**Cancer:** A malignant disease characterized by the abnormal growth and differentiation of cells. The product of neoplasia is a neoplasm (a tumor or cancer), which is an abnormal growth of tissue that results from excessive cell division. A tumor that does not metastasize is referred to as "benign." A tumor that invades the surrounding tissue and/or can metastasize is referred to as "malignant." Neoplasia is one example of a proliferative disorder. A "cancer cell" is a cell that is neoplastic, for example a cell or cell line isolated from a tumor.

Examples of solid tumors, such as sarcomas and carcinomas, and which include cancers of internal body cavities, include fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, lymphoid malignancy, pancreatic cancer, breast cancer, lung cancers (such as small cell lung carcinoma and non-small cell lung carcinoma), ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, melanoma, and CNS tumors (such as a glioma, astrocytoma, medulloblastoma, craniopharyogioma, ependymoma, pinealoma, hemangioblastoma, acoustic neuroma, oligodendroglioma, menangioma, neuroblastoma or retinoblastoma).

**Cell cycle:** The cycle of cellular division, which consists of M phase, G1 phase, S phase, and G2 phase. During S phase (synthesis), the cell replicates its DNA, doubling the cell DNA content. The period from the end of DNA replication until mitosis is G2 (gap 2) during which the cell nucleus contains two sets of chromosomes (also referred to as 4N state). M phase is mitosis, during which the duplicated chromosomes are separated and two daughter cells are formed. Once M phase is complete, the cell enters G1 phase (gap 1), during which RNAs and proteins are synthesized and cell size increases.

Progression through the cell cycle is tightly regulated by cell cycle checkpoints, control mechanisms that ensure that each phase of the cell cycle has been accurately completed prior to progression to the next phase. In particular, there are three major cell cycle checkpoints: G1/S checkpoint (also known as the "restriction point" in animal cells or the "start point" in yeast cells), G2/M checkpoint, and anaphase checkpoint.

Cells undergo "cell cycle arrest" when they are blocked from progression through the cell cycle. In a particular example, cells are arrested in the cell cycle prior to mitosis. "Cell cycle modulators" are agents that can synchronize and/or arrest cells at a particular cell cycle phase, such as at the G1 and S-phase or at any of the cell cycle checkpoints known in the art between phases.

**Chemotherapeutic agents:** Any chemical agent with therapeutic usefulness in the treatment of diseases characterized by abnormal cell growth. Such diseases include tumors, neoplasms, and cancer as well as diseases characterized by hyperplastic growth such as psoriasis. In one embodiment, a chemotherapeutic agent is an agent of use in treating a bladder cancer, a urinary cancer, or another tumor, such as an anti-neoplastic agent. In one embodiment, a chemotherapeutic agent is a radioactive compound. One of skill in the art can readily identify a chemotherapeutic agent of use (see for example, Slapak and Kufe, Principles of Cancer Therapy, Chapter 86 in Harrison's Principles of Internal Medicine, 14th edition; Perry et al., Chemotherapy, Ch. 17 in Abeloff, Clinical Oncology 2nd ed., 2000 Churchill Livingstone, Inc; Baltzer and Berkery. (eds): Oncology Pocket Guide to Chemotherapy, 2nd ed. St. Louis, Mosby-Year Book, 1995; Fischer Knobf, and Durivage (eds): The Cancer Chemotherapy Handbook, 4th ed. St. Louis, Mosby-Year Book, 1993). Common chemotherapeutic agents include mitomycin C, valrubicin, thiotepa, ethoglucid (epodyl), paclitaxel, pirarubicin, apaziquone, vicinium, interleukin-2, cisplatin, paclitaxel, docetaxel, doxorubicin, epirubicin, topotecan, irinotecan, mitomycin, iazofurine, gemcitabine, etoposide, vinorelbine, tamoxifen, valspodar, cyclophosphamide, methotrexate, fluorouracil, mitoxantrone, and vinorelbine. Combination chemotherapy is the administration of more than one agent to treat cancer.

**Contacting:** Placement in direct physical association. Includes both in solid and liquid form. Contacting can occur *in vitro* with isolated cells or *in vivo* by administering to a subject.

**Control:** A reference standard. In particular examples a control sample is taken from a subject that is known not to have a disease or condition. In other examples a control is taken from the subject being diagnosed, but at an earlier time point, either before disease onset or prior to or at an earlier time point in disease treatment.

**Effective amount of a compound:** A quantity of compound sufficient to achieve a desired effect in a subject being treated. An effective amount or "therapeutically effective amount" of a compound can be administered in a single dose, or in several doses, for example daily, during a course of treatment. However, the effective amount of the compound will be dependent on the compound applied, the subject being treated, the severity and type of the affliction, and the manner of administration of the compound.

**Intravesical instillation:** Also known as "intravesical therapy;" a medical procedure involving the direct administration of a drug into the urinary tract, either by retrograde or by antegrade route, comprising any parts such as: bladder, ureters, kidney pyelocalyceal system (kidney pelvis and calyces). Comparable drug administration is possible for other body cavities. In particular embodiments, intravesical instillation involves delivery of a drug through a catheter. In particular embodiments of the compositions and uses described herein, hydrogel-based compositions are provided to a subject by intravesical instillation.

**Pharmaceutically acceptable carriers:** The active agents for use in the described methods can be, mixed with a pharmaceutically acceptable carrier. The pharmaceutically acceptable carriers useful in this disclosure are conventional. Remington's Pharmaceutical Sciences, by E. W. Martin, Mack Publishing Co., Easton, PA, 15th Edition (1975), describes compositions and formulations suitable for pharmaceutical delivery of the compounds herein disclosed.

**Pharmaceutical agent:** A chemical compound or composition capable of inducing a desired therapeutic or prophylactic effect when properly administered to a subject or a cell. Incubating includes exposing a target to an agent for a sufficient period of time for the agent to interact with a cell. Contacting includes incubating an agent in solid or in liquid form with a cell.

**Sustained release composition:** A composition, typically matrix and/or polymeric based, that is capable of releasing a drug at a predetermined rate over an extended period of time by maintaining a relatively constant, effective drug level adjacent to or in the diseased tissue or organ. The thermoreversible hydrogels described herein are examples of sustained release compositions.

**Thermoreversible hydrogel:** The thermoreversible hydrogels for use in the described methods are in liquid form at room temperatures or below and remain liquid in the process of administration to a patient (*e.g*. through intravesical instillation). At elevated temperatures (*e.g*. typical human body temperature or lower), thermoreversible hydrogel solidifies, such as in a coating or filling of an internal body cavity.

**Urinary tract cancer:** Cancer of any area of the urinary tract, including the kidney (calyces and pelvis), ureter, bladder (also referred to as "urinary bladder"), and urethra. Common forms of bladder cancer include transitional cell carcinoma, inverted papilloma, and Squamous-cell carcinoma. Surgery followed by intravesical instillation of chemotherapy or BCG is a common treatment for urinary tract solid tumors.

**Wash:** Use of a fluid to cleanse an area. In particular embodiments, the "wash" of an area results in complete cleansing of the area. In other embodiments, washing an area does not produce complete cleansing. In still other embodiments, the "wash" can be a passive or natural wash such as but not limited to discharging of urine from the bladder, or an active process, that removes unwanted product by adding another product to clean the area. In particular embodiments of the described methods, a body cavity is "washed" between application of compositions containing a hydrogel and a therapeutic agent. In particular embodiments, the composition is completely washed out of the body cavity. In other embodiments, the composition is not completely washed out of the body cavity. The wash (and removal of fluid) of the methods described herein can be accomplished by standard methods of introducing and removing fluid from a body cavity.

### III. Overview of Aspects of the Invention

Provided herein are cell cycle modulators (CCMs) capable of arresting the cell cycle and/or synchronizing cells in the G1, S, G2 or M phase or between phases for the treatment of cancer of a body cavity, wherein the cell cycle modulator is administered in a first composition that includes, in addition to the CCM, a first sustained release composition, particularly a first thermoreversible hydrogel component that is administered locally to said body cavity, followed by administration to said body cavity of a second composition that includes at least one chemotherapeutic agent and a similar or different sustained release composition component, for example a thermoreversible hydrogel component.

A first aspect of the invention relates to the use of a cell cycle modulator capable of arresting the cell cycle in the S and/or G1 phase for the treatment of cancer of a body cavity. In particular embodiments the cancer of the body cavity is a urinary tract cancer, comprising but not limited to a bladder cancer or upper tract urothelial carcinoma (UTUC).

According to this aspect of the invention, the cell cycle modulator is administered comprised in a first composition comprising a first sustained release composition component, more particularly a thermoreversible hydrogel composition component, and said first composition is administered locally to said body cavity, and said administration of the first composition is followed by administration of a second composition comprising at least one chemotherapeutic drug or anti-cancer agent to said body cavity optionally embedded with second sustained release composition, for example a thermoreversible hydrogel composition.

In certain embodiments, the cell cycle modulator is administered comprised in a first composition comprising a first sustained release composition, and particularly a thermoreversible hydrogel composition, administered locally to said body cavity, followed by administration of a second composition comprising at least one anti-cancer agent to said body cavity

In certain embodiments, the anti-cancer agent is a small molecule pharmaceutical. In certain embodiments, the anti-cancer agent has a molecular mass of more than 160 u but less than 1000 u, particularly less than 700 u, more particularly less than 500 u, comprises up to five hydrogen bond donators (e.g., oxygen and or nitrogen atoms with one H attached), up to ten hydrogen bond acceptors (e.g., oxygen or nitrogen atoms) and is characterized by an octanol-water partition coefficient logP of below 5.6. These are the so-called "Lipinski" rules of 5 (originally, referring to molecules between 160 and 500 u) for drug-like compounds.

In certain embodiments, the cell cycle modulator is also capable of synchronizing cells.

In certain embodiments, the second composition comprises a second sustained release composition (e.g. thermoreversible hydrogel composition) component.

In certain embodiments, the second composition comprises a second sustained release composition, and particularly a thermoreversible hydrogel composition. In certain embodiments, the second composition is to be administered to the body cavity 2-196 hours, particularly 3 to 120 hours, more particularly 4 to 48 hours after the first composition is administered to the body cavity.

In certain embodiments, first composition is to be administered for at least 2 hours.

In a particular embodiment, the administration form comprising the CCM is to be washed from the subject prior to administration of the second composition. In certain particular embodiments, the second composition is to be administered to the body cavity 2-192 hours after the first composition is washed from the body cavity.

In certain particular embodiments, the second composition is to be administered to the body cavity 2-192 hours after administration of the first composition.

In some embodiments, the first composition is to be administered for at least 2 hours.

In certain embodiments, the concentration of the cell cycle modulator in the first composition is 0.5mmol/L- 1.8mol/L.

In certain embodiments, the concentration of the cell cycle modulator in the first composition ranges from 0.5mmol/L to 1.5 mol/L, particularly from 2 mmol/L to 250 mmol/L, more particularly from 10 mmol/L to 100 mmol/L.

In certain embodiments, the cell cycle modulator comprises hydroxyurea, alternol, resveratrol, fluorodeoxyuridine, thymidine dinucleotide pTpT, Zidovudine, PD-0332991 (Palbociclib, CAS No. 571190-30-2), LEE011, CINK4, Flavopiridol p276-00, alisertib, baraserib, danuserib, AT 9283, PF-03814735, AMG900), CHK1 and/or CHK2 inhibitors (UCN-1, XL-844, AZD7762, PF-477736), Polo-like kinases inhibitors (BI 2536, volasertib, GSK461364, MNN0905, Ro3280, SBE13HcL, NMSP 937, GW843682X) and/or P1446A-05.

In particular embodiments, the cell cycle modulator is selected from the group consisting of: hydroxyurea, alternol, resveratrol, fluorodeoxyuridine, thymidine dinucleotide pTpT and Zidovudine, PD-0332991, LEE011, CINK4, Flavopiridol, p276-00 and P1446A-05.

In certain embodiments, the anti-cancer agent is selected from the group consisting of: Mitomycin C, Apaziquone, Gemcitabine, Cisplatin, 5-FU (5- fluorouracil) Doxorubicin, Valrubicin, Epirubicin, Taxol, Pirarubicin and Methotrexate, and analogs thereof.

In certain embodiments, the first sustained release composition component, particularly, the first thermoreversible hydrogel component, is different from the second sustained release composition component, for example the second thermoreversible hydrogel component.

In certain embodiments, the first sustained release composition component, such as the first thermoreversible hydrogel component is the same as the second sustained release composition component, such as the second thermoreversible hydrogel component.

In yet another particular embodiment the second composition does not include any sustained release composition (*e.g.* thermoreversible hydrogel).

In certain embodiments, the cancer of the body cavity is a urinary tract cancer, particularly bladder cancer or upper tract urothelial carcinoma (UTUC).

In certain embodiments, the first and second compositions are to be administered to the urinary tract by instillation through the intravesical route.

In some embodiments, use of a first composition including CCM with or without hydrogel, is followed by radiotherapy, and further followed by local treatment with the second composition including a chemotherapeutic agent and sustained release composition (*e.g*. hydrogel).

Another aspect of the invention relates to a combination medicament comprising
- a first composition comprising a first sustained release composition component such as a thermoreversible hydrogel component and a cell cycle modulator capable of arresting the cell cycle in the S and/ or G1 phase, and
- a second composition comprising a second sustained release composition (*e*.*g*. thermoreversible hydrogel) comprising at least one chemotherapeutic agent.

In certain embodiments, the combination medicament is characterized by a concentration of the cell cycle modulator in the first composition that ranges from 0.5mmol/L to 1.5 mol/L, particularly from 2 mmol/L to 250 mmol/L, more particularly from 10 mmol/L to 100 mmol/L.

In certain embodiments, the cell cycle modulator is selected from the group consisting of: hydroxyurea, alternol, resveratrol, fluorodeoxyuridine, thymidine dinucleotide pTpT and Zidovudine, PD-0332991, LEE011, CINK4, Flavopiridol p276-00 and P1446A-05.

In certain embodiments, the chemotherapeutic agent is selected from the group consisting of: Mitomycin C, Apaziquone, Gemcitabine, Cisplatin, 5-FU (5-fluorouracil) Doxorubicin, Valrubicin, Epirubicin, Paclitaxel, Pirarubicin, Methotrexate and analogs thereof.

In certain embodiments of the described combination medicament, the thermoreversible hydrogel component of the first and/or second compositions includes or essentially consists of 20% to 40%, such as 32% or 35% (w/w) PEO/PPO block copolymer; and at least one mucoadhesive agent.

In particular embodiments, the first and/or second thermoreversible hydrogel component comprises or is essentially constituted of 20% to 35% (w/w) PEO/PPO block copolymer, for example 32% (w/w) PEO/PPO, and at least one mucoadhesive agent.

In certain embodiments, the mucoadhesive agent comprises crosslinked acrylic acid; polymethacrylates; divinyl glycol; polyethylene glycol; polyethylene oxide; vinylpyrrolidone/vinyl acetate copolymer; cellulose; microcrystalline cellulose; cellulose derivatives; gelatin; starch; starch derivatives; gums; dicalcium phosphate; lactose; sucrose; polyvinylpyrrolidone (PVP); polyvinyl alcohols (PVA); partially hydrolysed polyvinyl acetate (PVAc); polysaccharides; waxes; fats; fatty acid derivatives; hyaluronic acid; chitosan; •-carrageenan; carbomers; Eudragitor; and/or any combination thereof. In particular embodiments, the mucoadhesive agent is hydroxyl propylmethyl cellulose (HPMC) or PVP.

In certain embodiments, the combination medicament is administered locally to the body cavity, particularly to the bladder or upper urinary tract, for the treatment of bladder cancer and upper tract urothelial carcinoma, respectively.

In certain embodiments, the first and the second composition are administered by intravesical instillation.

In certain embodiments, the second composition is administered after the first composition has been washed from the body cavity.

In certain embodiments, the second composition is to be administered to the body cavity 2-168 hours after the first composition is administered to the body cavity. In certain embodiments, this interval is 3 to 120 hours. In certain particular embodiments, this interval is 4 to 48 hours.

In certain embodiments, the first composition is administered for at least 2 hours, particularly for at least 12 hours.

In some embodiments of the described combination medicament, the second composition is administered to the subject one, two, three or even four weeks after administration of the first composition.

In particular embodiments, the first sustained release composition (*e.g*. thermoreversible hydrogel composition) component is to be washed from the subject prior to administration of the second sustained release composition (*e.g*. thermoreversible hydrogel composition) component. In other particular embodiments of the described combination medicament, the second sustained release composition (*e.g*. thermoreversible hydrogel composition) component is to be administered to the body cavity 2-192 hours after the first component is washed from the body cavity. In yet other embodiments, the second composition is provided after a defined period of time, 1-4 weeks after the first composition is washed from the body cavity.

Another aspect of the invention relates to a packing unit comprising
- a first composition comprising a cell cycle modulator capable of arresting the cell cycle in the S phase and/or G1 phase, particularly a cell cycle modulator selected from hydroxyurea, alternol, resveratrol, fluorodeoxyuridine, thymidine dinucleotide pTpT, Zidovudine, PD-0332991, LEE011, CINK4, Flavopiridol p276-00 and P1446A-05, optionally with a sustained release composition component, such as a thermoreversible hydrogel component and;
- a second composition comprising a chemotherapeutic agent, particularly a chemotherapeutic agent selected from Mitomycin C, Apaziquone, Gemcitabine, Cisplatin, 5-FU (5- fluorouracil) Doxorubicin, Valrubicin, Epirubicin, Pirarubicin and Methotrexate optionally together with a sustained release composition, for example thermoreversible hydrogel, component.

In certain embodiments, the packing unit may further comprise a data sheet and/ user instructions.

In certain embodiments, the packaging unit's first and/or the second thermoreversible hydrogel components comprise:
- 20% to 35%, and more particularly 32% (w/w) PEO/PPO block copolymer and
- at least one mucoadhesive agent.

In certain embodiments, the concentration of the cell cycle modulator in the first composition ranges from 0.5mmol/L to 1.5 mol/L, particularly from 2 mmol/L to 250 mmol/L, more particularly from 10 mmol/L to 100 mmol/L.

In other particular embodiments, the chemotherapeutic agent is selected from the group consisting of: Mitomycin C, Apaziquone, Gemcitabine, Cisplatin, 5-FU (5-fluorouracil) Doxorubicin, Valrubicin, Epirubicin, Paclitaxel, Docetaxel, Cabazitaxel, Vinblastine, Vincristine, Pirarubicin, Methotrexate and analogs thereof.

The invention further relates to a packing unit that includes a first sustained release composition (*e.g*. thermoreversible hydrogel composition) composition including a cell cycle modulator capable of arresting the cell cycle in the G1, S and/or G2 phase or between phases, a second sustained release composition (*e.g*. thermoreversible hydrogel composition) composition including at least one chemotherapeutic agent, and instructions for preparation and use of the compositions in the packing unit.

### IV. Compositions for Sequential Sustained Treatment of a Body Cavity

Described herein are compositions for use in methods of treating a cancer found in or on the surface of an internal body cavity, such as a cavity of the urinary tract. The methods involve sequential administration of at least two compositions, the basis of one or both of which is a hydrogel with reverse thermal gelation properties, such that the gel is in solid gel form at an internal body temperature and in liquid form at reduced temperatures, such as any temperature below 18° C.

The first composition which is administered onto the surface of the internal body cavity is composed of a sustained release composition (*e.g*. thermoreversible hydrogel composition) combined with a cell cycle modulator (CCM), which synchronizes or arrests contacted cells at a particular phase of the cell cycle, such as but not limited to the G1 and S phases. Particular examples of cell cycle modulators include: CDKs (cyclin-dependent kinases) inhibitors and S phase arresting agents. Inhibitors of CDKs such as CDK1, 2, 4, or CDK 6 include but are not limited to PD-0332991, LEE011, CINK4, flavopiridol, p276-00 and P1446A-05. S phase arresting agents include small molecules such as but not limited to HU, Alternol, Resveratrol, Fluorodeoxyuridine, Thymidine dinucleotide pTpT and Zidovudine.

After a sufficient period of time for the CCM to take effect, a second composition is applied to the surface of the body cavity. The second composition can optionally include a sustained release composition (*e.g*. thermoreversible hydrogel composition) combined with an anti-cancer agent, such as a chemotherapeutic cytotoxic agent, or other antineoplastic agent (referred to collectively as "chemotherapeutic agents").

The localized, sequential, and sustained administration of the CCM- and chemotherapeutic-containing compositions provides an enhanced cytotoxic effect which more effectively treats the target cancer than any of the agents applied alone, whether or not administered in a sustained release composition.

In a particular embodiment, the use of radiotherapy is provided prior to or currently with the second composition, in order to increase the sensitivity of the targeted tumor cells to chemotherapy treatment.

In a particular embodiment, the body cavity which is contacted by the described compositions is any internal body cavity that produces and/or voids fluid in such a way that locally administered agents are quickly flushed from the site of administration. Particular examples of such cavities include the bladder, the upper urinary tract (including: the ureters, renal pelvis and calyces of one or both kidneys) abdomen, reproductive system (uterus, ovary cervix, vagina), nasal cavity, brain and the gastrointestinal tract.

In particular embodiments the cancer of the body cavity that can be treated with the described compositions is a solid cancer found on or in the surface of an internal body cavity. Particular non limiting examples include bladder cancer, urinary tract cancer, gastrointestinal cancer, vaginal cancer, uterine cancer, rectal cancer, thoracic cancer and pelvic cancer.

The compositions described herein are provided in a particular sequence, such that the composition containing the CCM ("first composition") is administered prior to the composition containing the chemotherapeutic agent ("second composition"). In a particular embodiment the second composition is administered 2-192, 2-144, 2-96, 2-72, 2-48, 2-36, 2-24 hours after the first composition, such as 2, 4, 6, 8, 10, 12, 14, 16, 18, 20, 24, 24-36, 36, 36-72, 12-72, 72, 72-96, 96, 96-120, 120, 120-144, 144, 144-168, 168, 168-192 or 192 hours after the first composition. In yet another alternative embodiment the first composition is administered one week to 4 weeks prior to the second composition.

Because the first and optionally the second compositions provide the active agents in a sustained release composition (*e.g*. thermoreversible hydrogel composition), the compositions can remain associated with the local area of administration for an extended period of time. In particular embodiments, the first and second compositions are allowed to remain in contact with the body cavity under treatment for the same amount of time. In other embodiments, the compositions remain in contact with the body cavity for different times. In particular embodiments, the first composition remains in contact with the body cavity for a longer period of time than the second composition. In other embodiments, the second composition remains in contact with the body cavity for a longer period of time than the first composition. In still other embodiments, the first composition is in contact with the body cavity for a specified period of time, whereas the second composition contacts the body cavity for an undefined period. The duration of treatment (and therefore of contact of each composition with the body cavity) in any of these embodiments can be hours or even days or weeks at a time.

In particular embodiments, either or both compositions can contact the body cavity for up to 60 hours, up to 48 hours, up to 36 hours, up to 24 hours, up to 22 hours, up to 20 hours, up to 18 hours, up to 16 hours, up to 14 hours, up to 12 hours, up to 10 hours, up to 8 hours, up to 4 hours, and up to 2 hours. In other embodiments, the compositions can contact the body cavity for up to 3, 4, 5, 6, 7 or more days.

In still other embodiments, the compositions can contact the body for at least a certain amount of time, such as at least 2-60 hours, such as at least 2, at least 4, at least 6, at least 8, at least 10, at least 12, at least 14, at least 16, at least 18, at least 20, at least 22, at least 24, at least 36, at least 48 hours, at least 60 hours or more.

In particular embodiments wherein one or more of the compositions contacts the body for a minimal amount of time (e.g. less than 4 hours, such as less than 3, 2, or 1 hour), the active ingredient (one or both of the CCM or the chemotherapeutic agent) need not be provided in a hydrogel composition, but may be administered locally, such as by instillation in water, as is known in the art. In such embodiments for example, the chemotherapeutic agent can be provided in solution by local administration (in solution, such as by instillation) after the cells have been synchronized by in-gel administration of the CCM.

The administration of the first and second compositions is sequential; one following the other. In particular embodiments, the second composition is applied to the body cavity after a sufficient time has elapsed such that the first composition has been fully degraded or washed and is no longer associated with the body cavity.

In particular embodiments, the second composition is applied to the body cavity after 192 hours, up to 168 hours, up to 144 hours, up to 120 hours, up to 96 hours, up to 72 hours, up to 48 hours, up to 36 hours, up to 24 hours, up to 22 hours, up to 20 hours, up to 18 hours, up to 16 hours, up to 14 hours, up to 12 hours, up to 10 hours, up to 8 hours, up to 4 hours, and up to 2 hours. In other embodiments, the second compositions can be applied 7, 14, 21, 28 or more days after the first composition.

In particular embodiment, the CCM is given systemically.

In other embodiments, the first composition is washed out of the body cavity either by urination or by active washing prior to administration of the second composition. Such active washes can be achieved with any biocompatible solution known to the art.

In particular embodiments, following the wash, dissolution, or degradation of the composition, the second composition can be immediately administered to the body cavity. In other embodiments, the second composition is only administered after a specified period of time has elapsed. In particular embodiments, the second composition is administered from 1-192 hours after the wash, dissolution, or degradation (and removal of the first composition from the body cavity). For example, the second composition can be administered 2-192 hours after the wash, such as 2, 4, 6, 8, 10, 12, 14, 16, 18, 20, 22, 24, 36, or 48, 96, 168, 192 hours after the wash or 7,14,21,28 days after the wash.

In particular embodiments, more than two compositions are applied sequentially to the subject. One of skill will appreciate that the time which each composition in such methods can be applied to the body cavity can be any of the times described herein. Likewise, a sequence of washing steps of equal or varying duration can be included in such methods between administrations of each composition.

In certain particular embodiments, the two compositions are applied together but dissolved sequentially.

In particular embodiments, after the specified period of time, the second (or last in the case of more than two compositions) composition is washed from the internal body cavity. The washing solution can be the same or different from a solution used to wash the first composition from the body cavity.

The described composition allow for sustained local administration of active pharmaceutical agents in sustained release compositions that include a gel having reverse thermal gelation properties (a "thermoreversible hydrogel"). In particular embodiments, the thermoreversible hydrogel composition includes one or more components including poloxamer and optionally at least one mucoadhesive agent such as, but not limited to a methylcellulose, hydroxypropylmethylcellulose, alginates, cellulose acetophathalate, carbopol, gellan gum, xyloglucan, pectin, chitosan, and any combination thereof.

In particular embodiments the gel component of the thermoreversible hydrogel compositions comprises 5%-45% (w/w) and ranges therein of the composition, such as 10%-35%, 20%-45%, 15%-35%, 20%-40%, 20%-35%, and 32%.

In particular embodiments the thermoreversible hydrogel comprises an A-B-A type polymer, wherein A or B is selected from PEG (polyethylene glycol), PLGA (poly(lactic-co-glycolic) acid, PLA(polylactic acid) and PPO (polypropylene oxide). More preferably, A is PEO and B is PPO compound or A-B-A is PEO (Poly(ethylene oxide)/PPO block copolymer.

In a particular embodiment the thermoreversible hydrogel composition comprises 5% to 45% (w/w), and any range therein including 20%-35%, and 32% (w/w), of a PEO/PPO block copolymer and comprises at least one of a mucoadhesive enhancing agent, gelation temperature controlling agent, pH controlling agent, absorption enhancer/ tight junction modifier / cell membrane permeability enhancer, or an agent to produce an exothermic reaction.

In particular embodiments, the mucoadhesive enhancing agent can include, but is not limited to, agarose, chitosan, gelatin, hyaluronic acid, carrageenan, pectin, sodium alginate, polyacrylic acids, polymers based on poly(meth)acrylic acid, carbopol, polycarbophil, polyacrylic acid, polyacrylates, copolymer of acrylic acid and polyethylene glycol, copolymer of methylvinyl ether and methacrylic acid, poly-2-hydroxyethylmethacrylate, copolymer of acrylic acid and ethylhexylacrylate, polymethacrylate, polyalkylcyanoacrylates: polyisobutylcyanoacrylate, polyisohexylcyanoacrylate, cellulose derivatives (for example methylcellulose (MC), hydroxy-propylcellulose (HPC), hydroxyl propylmethyl cellulose (HPMC), hydroxy ethyl cellulose, thiolated CMC other hydroxyalkylcelluloses and hydroxyalkylmethylcelluloses, carboxy-methylcelluloses (CMC) and salts thereof), polymethacrylates, starch or starch derivatives including starch phosphate esters at various degrees of substitution, as well as gums like guar gum, locust beam gum and xanthan gum and their derivatives. Polyvinylpyrrolidone (PVP) and its copolymers (N-vinyl-2-pyrrolidone), Poly-N-2-hydroxypropylmethacrylamide, polyhydroxyethylene, polyvinyl alchohol (PVA), and thiolated polymers.

In particular embodiments, gelation temperature controlling agents include, but are not limited to, urea, polyethylene glycol, short chain fatty acid and their salts (sodium octanoate, sodium dodecyl sulfate), ethanol, Glyceryl monostreatrate, Isopropyl myristate, and Polysorbate surfactants.

In some embodiments, tight junction modifier / cell membrane permeability enhancers include, but are not limited to, anionic surfactants, non-anionic surfactants, charged polymers, dimethyl sulfoxide (DMSO), decylmethyl sulfoxide, tert-butyl cyclohexanol, fatty acids their esters and salts, ethanol, nicotinamide, urea, perfluoropolyether, monoterpene ketones, disodium citrate, succinic acid, alkyl saccharides, hyaluronidase and tris.

In another embodiment, the thermoreversible hydrogel comprises between 10% and 40% (w/w), particularly 20%-35%, and 32% (w/w) PEO/PPO block copolymer and optionally between 0.05% and 2.5% (w/w) mucoadhesive agent.

In other embodiments the thermoreversible hydrogel comprises between 20% and 30% (w/w) PEO/PPO block copolymer and between 0.05% and 0.5% (w/w) hydroxypropylmethylcellulose (HPMC) or Polyvinylpyrrolidone (PVP) and between 0.01% and 2.5% (w/w) polyethylene glycol (PEG)-400; and the balance water.

In particular embodiments, the sustained release composition (*e.g*. thermoreversible hydrogel composition) of the first and second compositions is the same. In other embodiments, the sustained release composition (*e.g*. thermoreversible hydrogel composition) is different in the different compositions. For example, in some embodiments, the thermoreversible hydrogel composition of the first composition degrades more quickly than that of the second composition under physiological conditions. In other embodiments, the thermoreversible hydrogel composition of the second composition degrades more quickly than that of the first composition under physiological conditions.

The gelation properties of the thermoreversible hydrogel composition allow the first and second compositions to be administered in liquid form to the surface of an internal body cavity. Such administration can be by any method known to the art, using any device suitable to apply the subject compositions. For example, methods known to the art of intravesical instillation of an agent can be used to administer the compositions described herein to the surface of an internal body cavity.

As described herein, a CCM is administered to an internal body cavity in a first composition. The results presented herein demonstrate that application of a CCM to urothelial cancer cells for a sustained period of time can synchronize a majority of the cells in a sample at a particular phase of the cell cycle, such as but not limited to the S or G1 phase. In particular embodiments of the described methods, the CCM can be an agent that be an S-phase or G1-phase arresting agent, including, but not limited to, CDK4/6 inhibitors (such as, but not limited to, PD-0332991, LEE011, CINK4, flavopiridol p276-00 and P1446A-05), and to small molecules such as HU, alternol, resveratrol, fluorodeoxyuridine, thymidine dinucleotide pTpT and Zidovudine. Such molecules are known in the art to have a cytotoxic effect in addition to synchronizing cells in the S phase and/or G1-phase. For example, HU is an antineoplastic agent commonly used to treat myeloproliferative disorders and other non-neoplastic conditions. Despite an intrinsic cytotoxic effect, it is shown herein that when administered sequentially with a second chemotherapeutic agent, not only are the cytotoxic effects significantly enhanced, but the dosage of the second agent needed for comparable effects when administered alone can be significantly reduced.

In particular embodiments, the CCM is provided in the first composition at a concentration of 0.5 mM-1.5M, such as but not limited to about 1-5 mM, 5-10 mM, 10-15 mM, 15-20 mM, 20-25 mM, 25-30 mM, 30-35 mM, 35-40 mM, 40-45 mM, 45-50 mM, 1 mM, 5 mM, 10 mM, 20 mM or 25 mM.

The second composition as described herein includes at least one anti-cancer agent such as but not limited to a chemotherapeutic cytotoxic, anti-neoplastic, or anti-angiogenic agent. Examples of such agents for use in the described methods include, but are not limited to MMC, apaziquone, BMS-181174, gemcitabine, cisplatin, 5-FU (5- fluorouracil) Doxorubicin, Valrubicin, Epirubicin, Pirarubicin, Methotrexate and analogs thereof. It will be appreciated that the anti-cancer agent can be for example any known chemotherapeutic agent known in the art that is suitable for treatment of the cancer to be treated by the subject method. In particular examples, the agent is a small molecule agent. In other examples, the agent is a biological agent such as an antibody, protein, or nucleic acid based agent. Particular examples of nucleic acid-based agents used in anti-cancer therapies include nucleic acids for use in gene silencing (miRNAs, siRNAs, and the like), as DNA vectors expressing therapeutic RNAs and proteins.

As described herein, use of the described compositions also allows for use of a lower concentration of chemotherapeutic agent than might otherwise be needed to produce a similar effect for treatment of a cancer of an internal body cavity. As a result, 2, 3, 4, 5, 6, 7, 8, 9, 10 fold or less chemotherapeutic agent is needed in the second composition than might be needed to provide a similar cytotoxic therapeutic effect if the compound was not administered as described herein. It will be appreciated that different chemotherapeutic agents will have different dosing regimens depending on a particular subject, the cancer type, and their individual treatment needs. However, the surprising results provided herein allow for highly efficacious treatment simultaneous with decreased medication requirements.

The compositions herein are for use in treatment of a cancer of an internal body cavity, particularly of the urinary tract, including one or both kidneys, ureters, the bladder, or urethra of a subject. Non-limiting examples of such cancers include urinary tract cancer, particularly bladder cancer or UTUC.

The compositions described herein can be provided as a combination medicament that includes a first sustained release composition (*e.g*. thermoreversible hydrogel composition) component including a cell cycle modulator (CCM) capable of arresting the cell cycle in the S or G1 phase, and a second sustained release composition (*e.g*. thermoreversible hydrogel composition) component including at least one chemotherapeutic agent. Aspects of the CCM, hydrogel components, and chemotherapeutic agent are as described above. Likewise, the uses, sequential administration, and target cancers for the described combination medicament are as described above.

The compositions described herein, in all of the described embodiments, can be supplied in packing units or kits that include the described first sustained release composition (*e.g*. thermoreversible hydrogel composition) composition including a cell cycle modulator capable of arresting the cell cycle in the S phase, the second sustained release composition (*e.g*. thermoreversible hydrogel composition) composition including at least one chemotherapeutic agent, and instructions for preparation and use of the compositions in the packing unit or kit.

The following items relate to particular non-limiting embodiments of the invention.
Item 1: A cell cycle modulator capable of arresting the cell cycle in the S and/or G1 phase for the treatment of cancer of a body cavity,
   wherein the cell cycle modulator is administered comprised in a first composition comprising a first sustained release composition (*e.g*. thermoreversible hydrogel composition) and said first composition is administered locally to said body cavity,
   and wherein said administration of the first composition is followed by administration of a second composition comprising at least one chemotherapeutic or anti-cancer agent to said body cavity.
Item 2: The cell cycle modulator for the treatment of cancer of a body cavity according to item 1, wherein the cell cycle modulator is also capable of synchronizing cells.
Item 3: The cell cycle modulator for the treatment of cancer of a body cavity according to item 1 or 2, wherein the second composition comprises a second sustained release composition (*e.g*. thermoreversible hydrogel composition) component.
Item 4: The cell cycle modulator capable for the treatment of cancer of a body cavity according to any one of items 1 to 3, wherein the second composition is to be administered to the body cavity 2-196 hours, particularly 3 to 120 hours, 4 to 96, 8 to 36, 12 to 24, and more particularly 4 to 48 hours after the first composition is administered to the body cavity.
Item 5: The cell cycle modulator capable for the treatment of cancer of a body cavity according to any one of the preceding items, wherein said first composition is to be administered for at least 2 hours.
Item 6: The cell cycle modulator capable for the treatment of cancer of a body cavity according to any one of the preceding items, wherein the concentration of the cell cycle modulator in the first composition ranges from 0.5mmol/L to 1.5 mol/L, particularly from 0.5mmol/L to 1.5 mol/L, particularly from 2 mmol/L to 250 mmol/L, more particularly from 10 mmol/L to 100 mmol/L.
Item 7: The cell cycle modulator capable for the treatment of cancer of a body cavity according to any one of the preceding items, wherein the cell cycle modulator is selected from the group consisting of: hydroxyurea, alternol, resveratrol, fluorodeoxyuridine, thymidine dinucleotide pTpT and Zidovudine, PD-0332991, LEE011, CINK4, Flavopiridol, p276-00 and P1446A-05.
Item 8: The cell cycle modulator capable for the treatment of cancer of a body cavity according to any one of the preceding items, wherein the anti-cancer agent is selected from the group consisting of: Mitomycin C, Apaziquone, Gemcitabine, Cisplatin, 5-FU (5- fluorouracil) Doxorubicin, Valrubicin, Epirubicin, Taxol, Pirarubicin and Methotrexate. and analogs and/or functional derivatives thereof.
Item 9: The cell cycle modulator capable for the treatment of cancer of a body cavity according to any one of the preceding items 3 to 8, wherein the first sustained release composition (*e.g*. thermoreversible hydrogel composition) component is different from the second sustained release composition (*e.g*. thermoreversible hydrogel composition) component.
Item 10: The cell cycle modulator capable for the treatment of cancer of a body cavity according to any one of the preceding items, wherein said first sustained release composition (*e.g*. thermoreversible hydrogel composition) component is the same as the second sustained release composition (*e.g*. thermoreversible hydrogel composition) component.
Item 11: The cell cycle modulator capable for the treatment of cancer of a body cavity according to any one of the preceding items, wherein the cancer of the body cavity is a urinary tract cancer, particularly bladder cancer or upper tract urothelial carcinoma (UTUC).
Item 12: The cell cycle modulator capable for the treatment of cancer of a body cavity, particularly the urinary tract, according to any one of the preceding items, wherein the first and second compositions are to be administered to the urinary tract by instillation through the intravesical route.
Item 13: A combination medicament comprising
   - a first composition comprising a first sustained release composition (*e.g*. thermoreversible hydrogel composition) component and a cell cycle modulator capable of arresting the cell cycle in the S and/ or G1 phase, and
   - a second composition comprising a second the sustained release composition (*e.g*. thermoreversible hydrogel composition) component comprising at least one chemotherapeutic agent.
Item 14: The combination medicament according to item 13, wherein the concentration of the cell cycle modulator in the first composition ranges from 0.5mmol/L to 1.5 mol/L, particularly from 2 mmol/L to 250 mmol/L, more particularly from 10 mmol/L to 100 mmol/L.
Item 15: The combination medicament according to item 13 or 14, wherein the cell cycle modulator is selected from the group consisting of: hydroxyurea, alternol, resveratrol, fluorodeoxyuridine, thymidine dinucleotide pTpT and Zidovudine, PD-0332991, LEE011, CINK4, Flavopiridol p276-00 and P1446A-05.
Item 16: The combination medicament according to any one of items 13 to 15, wherein the chemotherapeutic agent is selected from the group consisting of: Mitomycin C, Apaziquone, Gemcitabine, Cisplatin, 5-FU (5- fluorouracil) Doxorubicin, Valrubicin, Epirubicin, Paclitaxel, Pirarubicin, Methotrexate and analogs thereof.
Item 17: The combination medicament according to any one of items 13 to 16, wherein the first and/or second thermoreversible hydrogel component comprises 20% to 40%, and particularly 20%-35%, or 32% (w/w) PEO/PPO block copolymer;
   - 0.05% to 0.5% (w/w) hydroxypropylmethylcellulose (HPMC) or Polyvinylpyrrolidone (PVP);
   - 0.1% to 2.5% (w/w) polyethylene glycol (PEG)-400;
   - and the balance is water.
Item 18: The combination medicament according to any one of items 13 to 16, wherein the first and/or second thermoreversible hydrogel component comprises or is essentially constituted of:
   - 20% to 30% (w/w) PEO/PPO block copolymer and
   - at least one mucoadhesive agent, particularly a mucoadhesive agent selected from HPMC and PVP.
Item 19: The combination medicament according to any one of items 13 to 18 for the treatment of cancer of a body cavity, particularly for the treatment of urinary tract cancer, more particularly for the treatment of bladder cancer and upper tract urothelial carcinoma by local administration to said body cavity.
Item 20: The combination medicament for the treatment of cancer of a body cavity according to item 19, wherein the first and the second composition are administered by intravesical instillation.
Item 21: The combination medicament for the treatment of cancer of a body cavity according to any of the previous items, wherein the second composition is administered after the first composition has been washed from the body cavity.
Item 22: The combination medicament for the treatment of cancer of a body cavity according to any one of items 13 to 21, wherein the second composition is to be administered to the body cavity 2-168 hours after the first composition is administered to the body cavity.
Item 23: The combination medicament for the treatment of cancer of a body cavity according to any one of items 13 to 21 wherein said first composition is administered for at least 2 hours, particularly for at least 12 hours.
Item 24: A packing unit comprising
   a first composition comprising a first sustained release composition (*e.g*. thermoreversible hydrogel composition) component and a cell cycle modulator capable of arresting the cell cycle in the S phase and/or G1 phase, particularly a cell cycle modulator selected from hydroxyurea, alternol, resveratrol, fluorodeoxyuridine, thymidine dinucleotide pTpT, Zidovudine, PD-0332991, LEE011, CINK4, Flavopiridol p276-00 and P1446A-05;
   a second composition comprising a second the sustained release composition (*e.g*. thermoreversible hydrogel composition) component and at least one chemotherapeutic agent, particularly a chemotherapeutic agent selected from Mitomycin C, Apaziquone, Gemcitabine, Cisplatin, 5-FU (5- fluorouracil) Doxorubicin, Valrubicin, Epirubicin, Pirarubicin and Methotrexate.
Item 25: The packaging unit according to item 24, wherein said first and/or the second sustained release composition (*e.g*. thermoreversible hydrogel composition) components comprise:
   - 20% to 35%, 30%, 32%, or 35% (w/w) PEO/PPO block copolymer and
   - at least one mucoadhesive agent.
Item 26: The packaging unit according to item 24 or 25, wherein the concentration of the cell cycle modulator in the first composition ranges from 0.5mmol/L to 1.5 mol/L, particularly from 0.5mmol/L to 1.5 mol/L, particularly from 2 mmol/L to 250 mmol/L, more particularly from 10 mmol/L to 100 mmol/L.

The following figures and examples are provided to illustrate certain particular features and/or embodiments. These examples should not be construed to limit the disclosure to the particular features or embodiments described.

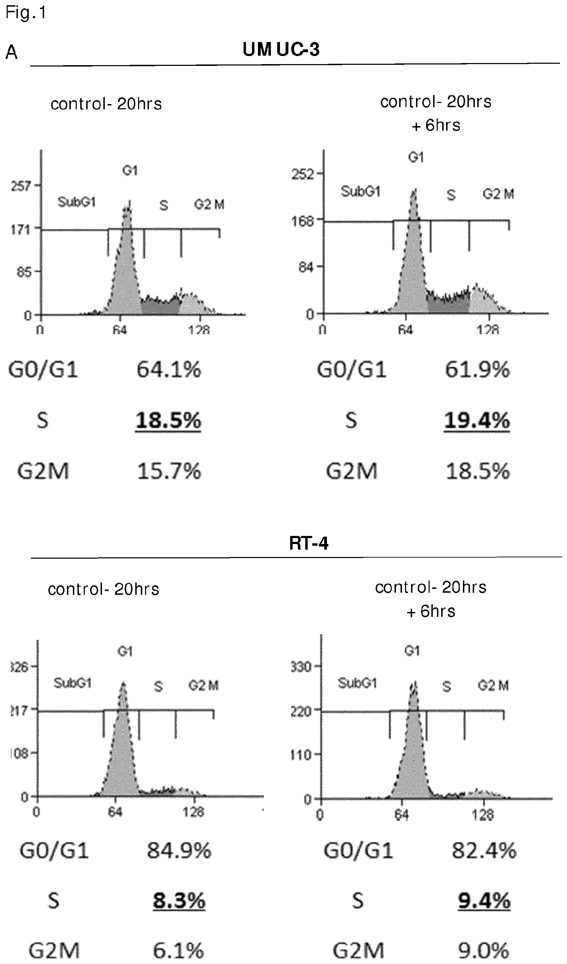

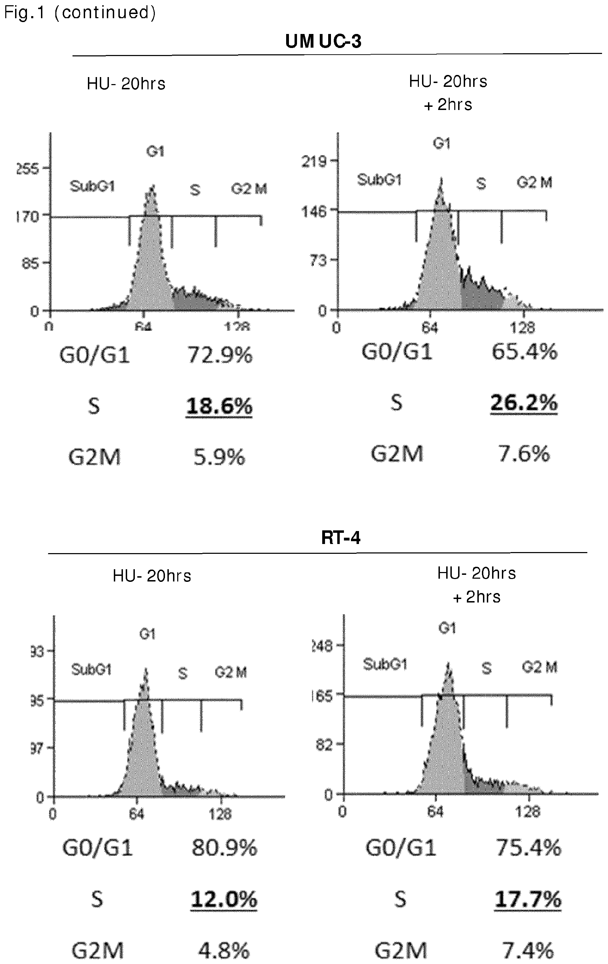

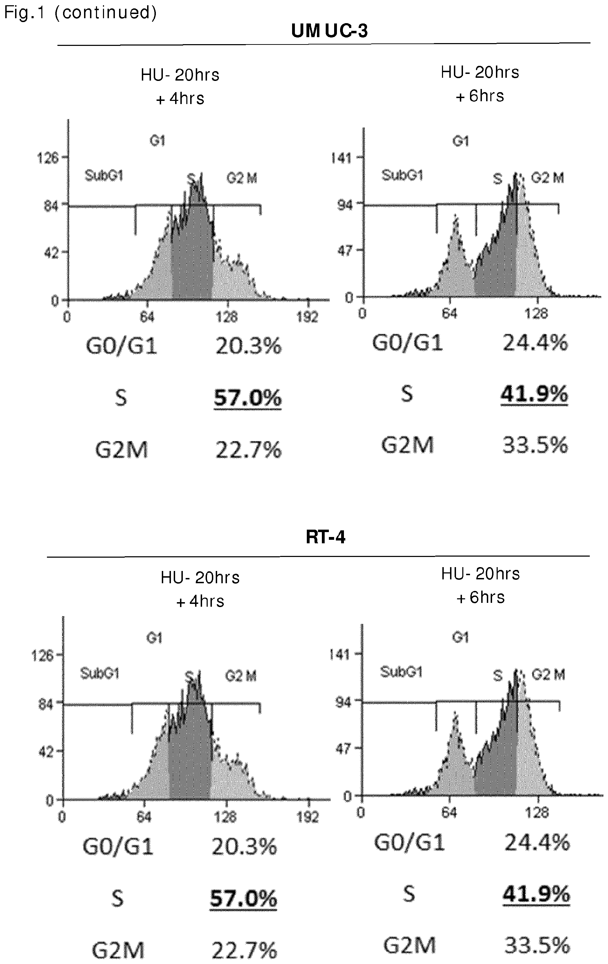

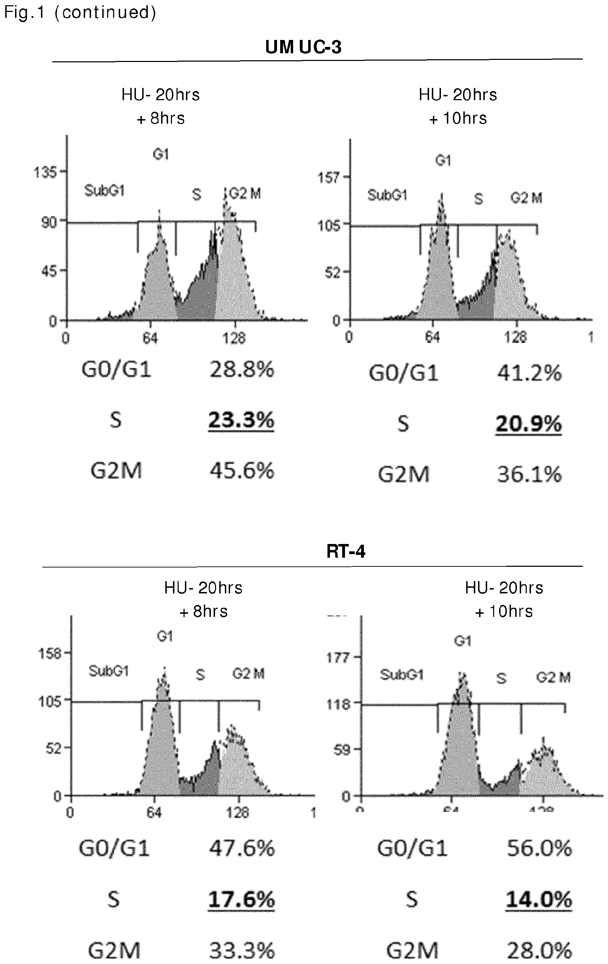

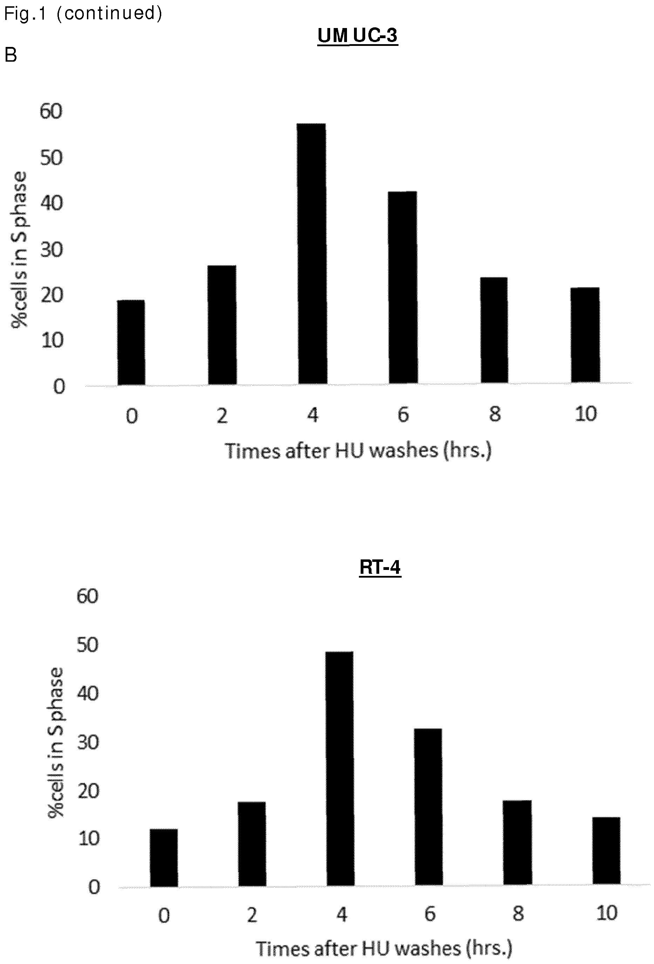

**Figures 1A** **and** **1B** **show cell cycle synchronization at the S phase following HU exposure in UMUC-3 and RT-4 cells.** **Figure 1A****:** UMUC-3 and RT-4 cells were cultured with 1mM hydroxyurea (HU) or with DMSO (control) for 20 hours. Then, the HU (or DMSO) was washed from the cells and replaced by fresh media. Cells were fixed and labeled with PI immediately or between 2 to 10 hours following HU washes. Cell cycle analysis was done by flow cytometry. The data indicate percentage of cells in each cell cycle phase and the percentage of cells at the S phase is underlined. **Figure 1B****:** A graphical representation of the percentage of cells in S phase, at the indicated times following washing, is shown for both UMUC-3 and RT-4 cell lines.

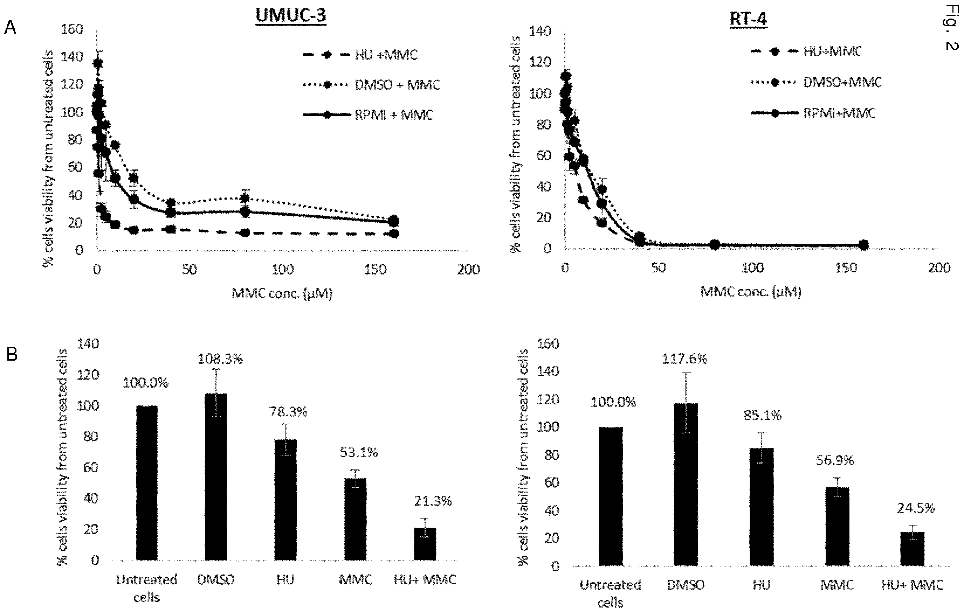

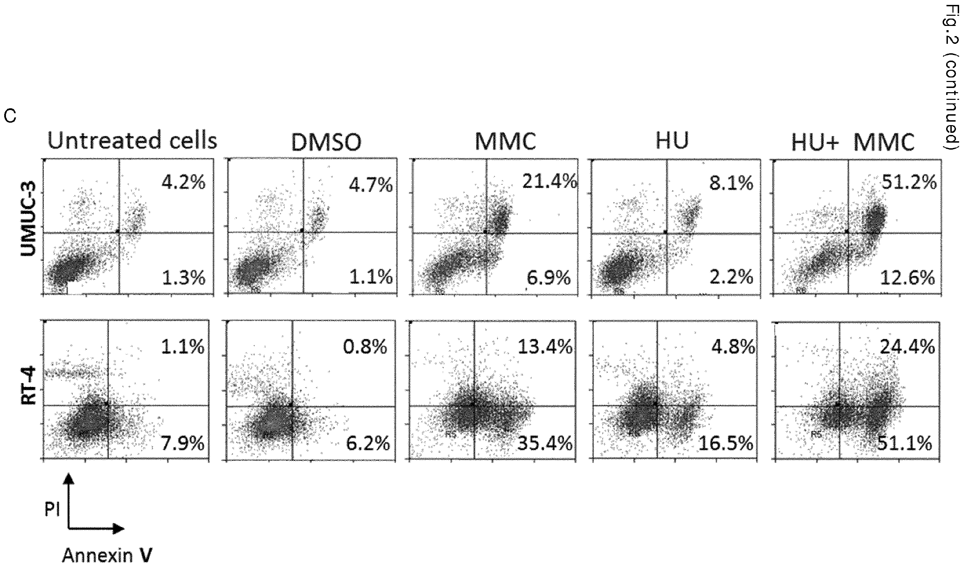

**Figures 2A-2C** **show viability and cytotoxicity of UMUC-3 and RT-4 cells following combined treatment with HU followed by MMC.** **Figure 2A****:** UMUC-3 and RT-4 cells were pretreated with 1mM HU (or DMSO/ RPMI only as a control) for 20 hours and then washed from the cells with fresh media. Two hours following washes, cells were treated with different MMC concentrations (0-160• M) for 8 hours. Cell viability analyzed 48 hours following HU washes. Shown are the results of XTT assays as determined in an ELISA reader at 490nm. The percentage of viable cells was calculated from untreated cells (viability of 100%). **Figure 2B** shows the percentage differences in viability between untreated cells (RPMI), cells treated with DMSO only, cells treated with only one drug (HU / MMC) and cells treated with HU+ MMC. Shown are averages of five (UMUC-3 cells) or four (RT-4 cells) independent experiments. These are calculated from the MMC EC₅₀ concentration values (10µM- UMUC-3 cells, 5-20 µM- RT-4 cells). **Figure 2C****:** Cytotoxicity percentages following treatment with HU/DMSO alone, MMC alone, and HU followed by MMC in UMUC-3 and RT-4 cells. Cells were treated with 1mM HU/DMSO or left untreated for 20 hours, and then washed. Two hours following HU/DMSO withdrawal, 10µM MMC (or RPMI media to the control samples: untreated cells, DMSO and HU alone) were added for additional 8 hours followed by washes. 48 hours after HU/DMSO washes, cells were stained with Annexin V FITC and PI and double positive cells were analyzed by flow cytometer (Cyan) using a single laser emitting excitation light at 488 nm. Results are presented in a logarithmic display. The X-axis reflects log Annexin V-FITC fluorescence intensity and the Y-axis reflects the propidium iodide fluorescence intensity. Data are displayed on a four-decade log scale, so the range is from 10° to 10⁴ (10⁰,10¹,10²,10³,10⁴). The number of gates is the following: In the first row: upper left field R4, upper right field R5, lower left field R6 and lower right field R7. In the second row: upper left field R3, upper right field R4, lower left field R5 and lower right field R6.

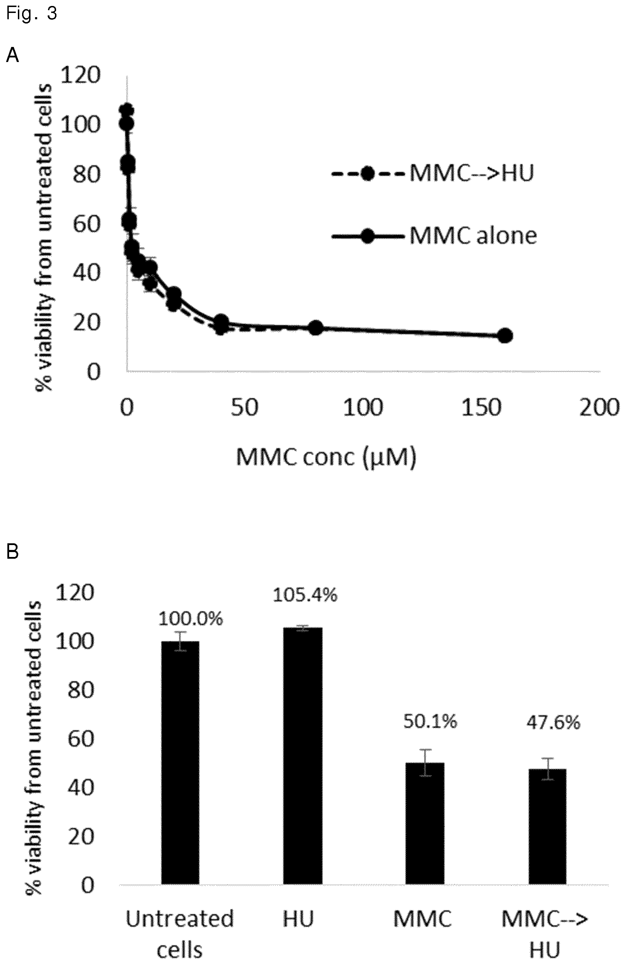

**Figures 3A and 3B** **demonstrate viability percentages following opposite sequential treatment (MMC followed by HU) in UMUC-3 cells.** **Figure 3A****:** UMUC-3 cells were pretreated by increased MMC concentrations for 8 hours followed by washes. Two hours following washes, cells were treated with 1mM HU (or RPMI only as a control [MMC alone]) for additional 20 hours. Cells were washed and cell viability analyzed 48 hours following HU washes. Shown are the results of XTT assays as determined in an ELISA reader at 490nm. The percentage of viable cells was calculated from untreated cells (viability of 100%). **Figure 3B** represents the viability percentages of the comparison between treatment with MMC alone at the EC50 value (calculated from figure 3A) to this opposite treatment- MMC (in the same concentration) followed by HU.

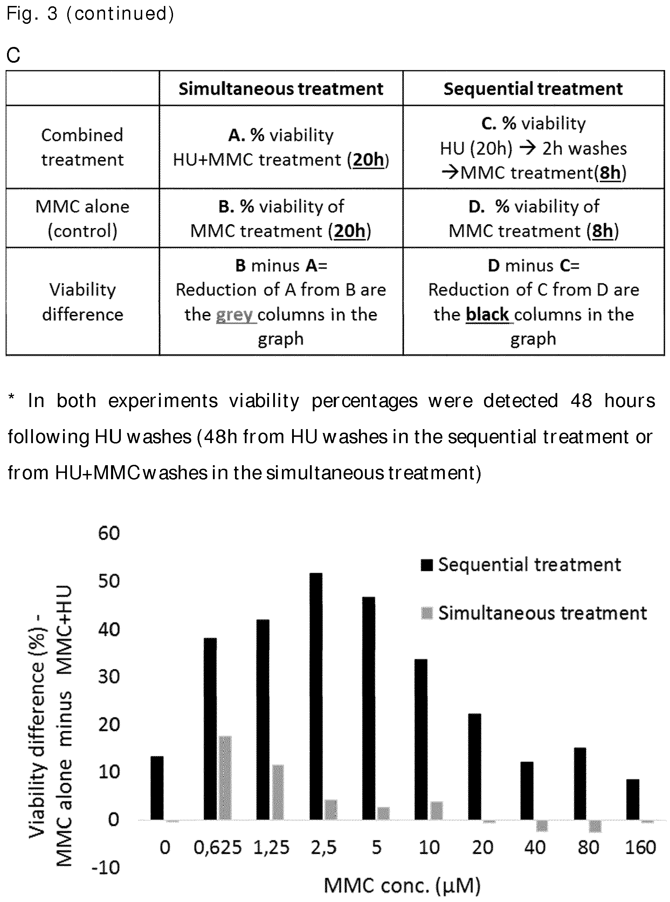

**Figure 3C** **shows a comparison of the percentage viability difference between sequential (HU followed by MMC) and simultaneous treatment in UMUC-3 cells.** The top panel is a table outlining the experimental methodology. The bottom panel is a graph comparing the difference in viability between sequential treatment (black bars of each pair) and simultaneous treatment (grey bars of each pair). In the simultaneous treatment, cells were treated simultaneously with HU (1mM) and MMC (increased concentrations), or only with MMC as a control (same concentrations), for 20 hours. In the sequential treatment, cells were pretreated with HU (1mM) followed by washes and 2 hours later, addition of MMC (increased concentrations) for 8 hours (the control was cells that were treated with MMC alone for 8 hours). For both experiments viability percentages were detected 48 hours following HU washes (48h from HU washes in the sequential treatment or from HU+MMC washes in the simultaneous treatment). The comparison of the percentages of viability difference were performed by reduction the viability values percentages of MMC+HU (sequential or simultaneous treatment) from the viability values of MMC alone treatment (the control for each experiment, respectively).

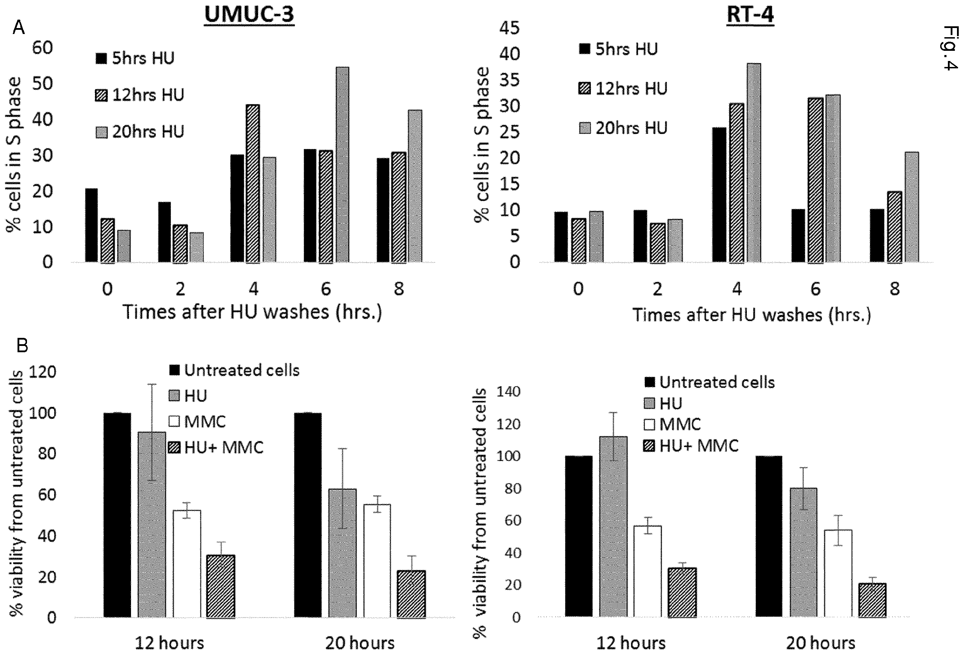

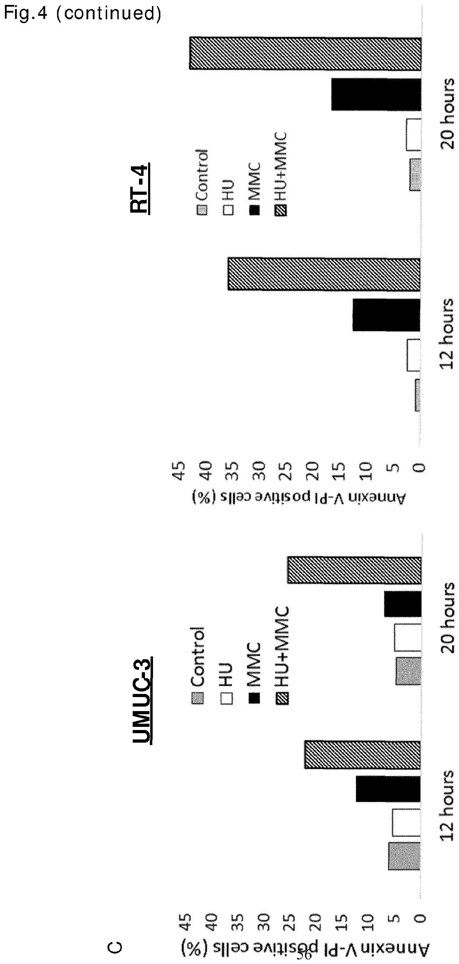

**Figures 4A-4C** **show exposure of UMUC-3 and RT-4 cells to HU for different time periods.** **Figure 4A****:** Cell cycle distribution of UMUC-3 and RT-4 cells following 5, 12, and 20 hour exposure to HU. Cells were cultured with 1mM HU for 5, 12, and 20 hours. Then, cells were fixed (0 hours) or washed and replaced by fresh media for 2, 4, 6, and 8 hours followed by fixation. Cells were labeled with PI and cell cycle analysis was done by flow cytometry. S phase percentages are presented in the graphs for both UMUC-3 and RT-4 cells. Cell viability (**Figure 4B**) and cell cytotoxicity (**Figure 4C**) of UMUC-3 and RT-4 cells pretreated with 1mM HU for 12 and 20 hours followed by MMC for 8 hours (MMC at the EC₅₀ concentration value) are presented. Viability and cytotoxicity were analyzed 48 hours following HU washes by XTT assay (ELISA reader at wave length of 490nm) or by staining with Annexin V FITC and PI (FACS) respectively.

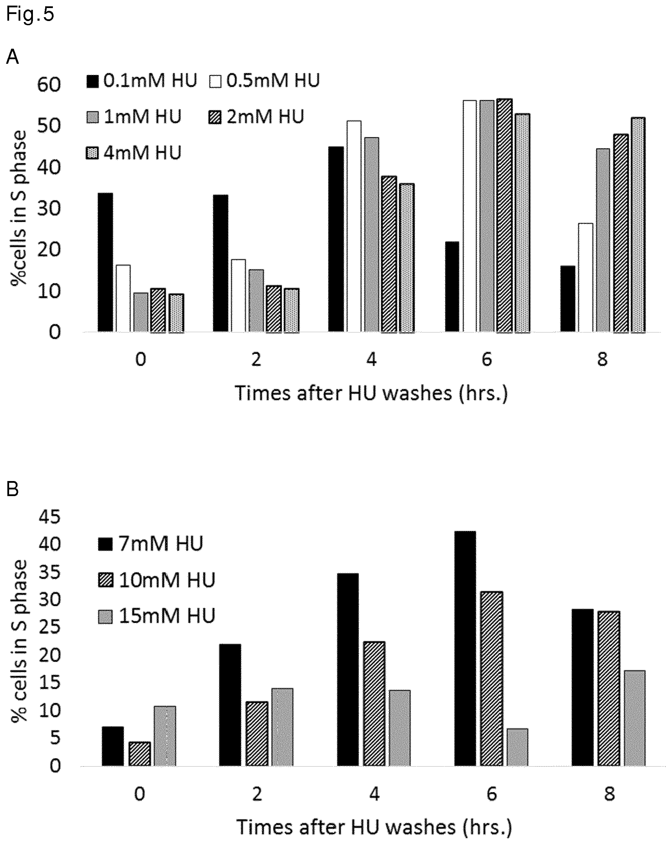

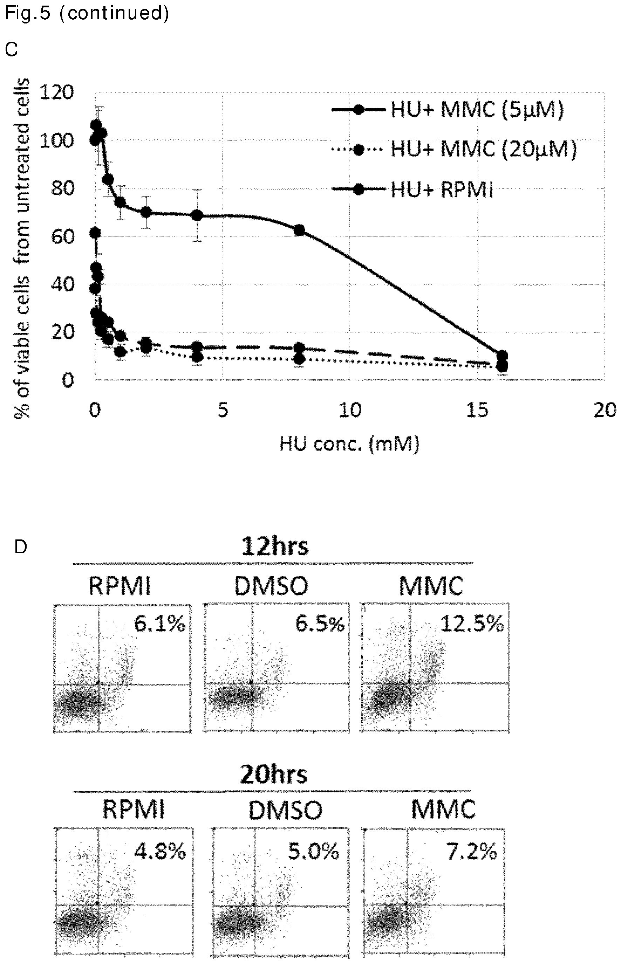

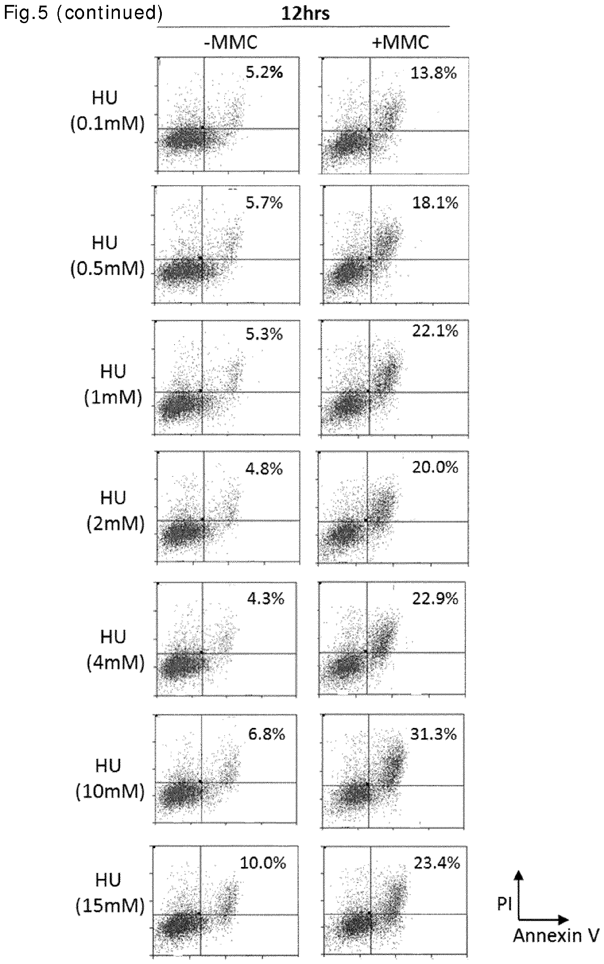

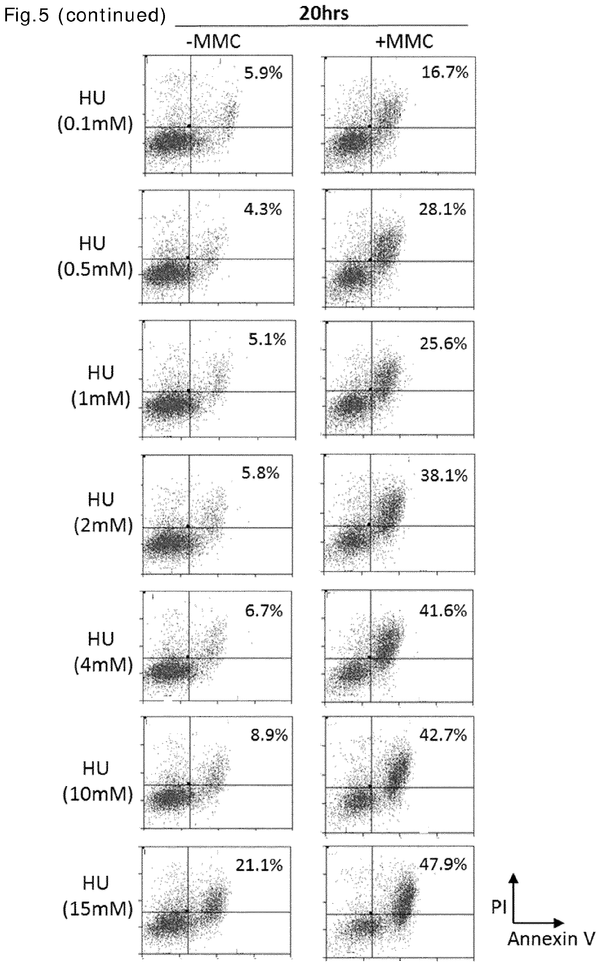

**Figures 5A-5D** **show treatment of UMUC-3 and RT-4 cells with several HU concentrations.** Cell cycle distribution of UMUC-3 cells following treatment with 0.1-4mM (**Figure 5A**) and 7-15mM (**Figure 5B**) of HU for 20 hours. Cells were fixed or washed and replaced by fresh media for 2, 4, 6, and 8 hours followed by fixation. Cells were labeled with PI and cell cycle analysis was done by flow cytometry. The graphs represent the S phase percentages following every treatment. **Figure 5C****:** Viability of UMUC-3 cells treated with various HU concentrations followed by MMC. UMUC-3 cells were pretreated with 0-16mM HU for 20 hours and washed. 2 hours following washes, cells were treated with 5µM or 20µM MMC for 8 hours. Cell viability was analyzed 48 hours after HU washes by XTT assay in an ELISA reader at wave length of 490nm. The percentage of viable cells was calculated from untreated cells (viability of 100%). Figure 5D: Cytotoxicity of UMUC-3 cells that were treated with 0.1-15mM HU /control (DMSO or without treatment [RPMI]) for 12 or 20 hours followed by washes and addition of 20µM MMC after 2 hours for additional 8 hours. After 48 hours from HU washes, cells were stained with Annexin V FITC and PI and double positive cells were analyzed by flow cytometer (Cyan) using a single laser emitting excitation light at 488nm. Results are presented in a logarithmic display. The X-axis reflects log Annexin V-FITC fluorescence intensity and the Y-axis reflects the propidium iodide fluorescence intensity. Data are displayed on a four-decade log scale, so the range is from 10° to 10⁴ (10⁰,10¹,10²,10³,10⁴). The number of gates in both rows is the following: upper left field R4, upper right field R5, lower left field R6 and lower right field R7.

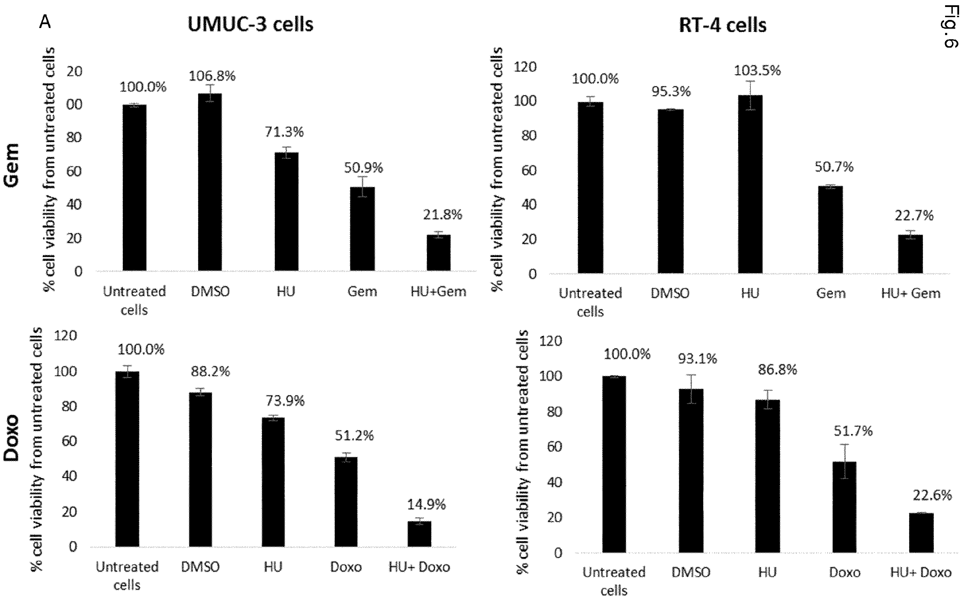

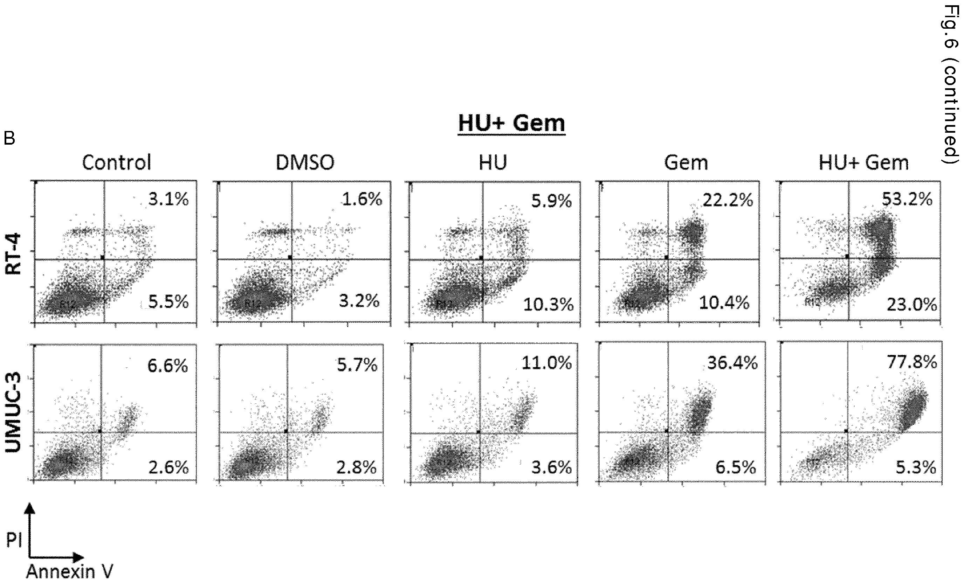

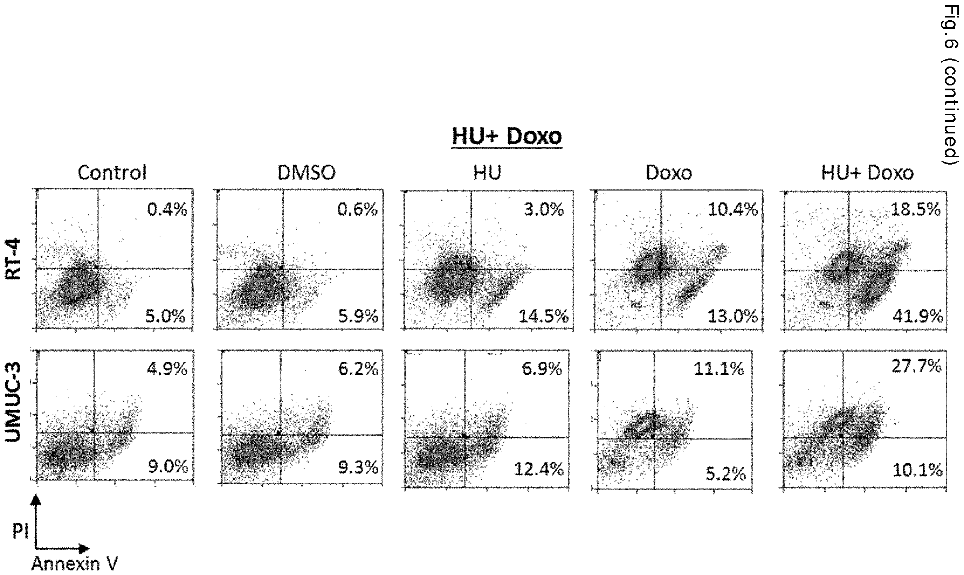

**Figures 6A** **and** **6B** **show enhancement of Gemcitabine and Doxorubicin activity following pretreatment with HU.** **Figure 6A****:** UMUC-3 and RT-4 cells were pretreated with 1mM HU (or DMSO control) for 20 hours and washed with clean media. Two hours following washes, cells were treated with either Gemcitabine (Gem) or Doxorubicin (Doxo) for 8 hours and washed. Cell viability was analyzed 24-48 hours following HU washes by XTT assay in an ELISA reader at wave length of 490nm. The percentage of viable cells was calculated from untreated cells (viability of 100%). Data shown are representative of three independent experiments for each cell line and each drug treatment and calculated from the EC₅₀ values. **Figure 6B****:** Cytotoxicity of UMUC-3 and RT-4 cells that were pretreated with 1mM HU /control (DMSO or without treatment) for 20 hours followed by washes and addition of Doxo/Gem (Doxo: 2.5µM in RT-4 cells and 5µM in UMUC-3 cells, Gem: 5µM in RT-4 cells and 0.5µM in UMUC-3 cells) after 2 hours for additional 8 hours. After 24 hours (for Doxo) or 72 hours (for Gem) from HU washes, cells were stained with Annexin V FITC and PI and double positive cells were analyzed by flow cytometer (Cyan) using a single laser emitting excitation light at 488nm. Results are presented in a logarithmic display. The X-axis reflects log Annexin V-FITC fluorescence intensity and the Y-axis reflects the propidium iodide fluorescence intensity. Data are displayed on a four-decade log scale, so the range is from 10° to 10⁴ (10⁰,10¹,10²,10³,10⁴). The number of gates in both rows is the following: upper left field R10, upper right field R11, lower left field R12 and lower right field R13. Figure 6B (continued): the number of gates is the following: in the first row: upper left field R3, upper right field R4, lower left field R5 and lower right field R6. In the second row: upper left field R10, upper right field R11, lower left field R12 and lower right field R13.

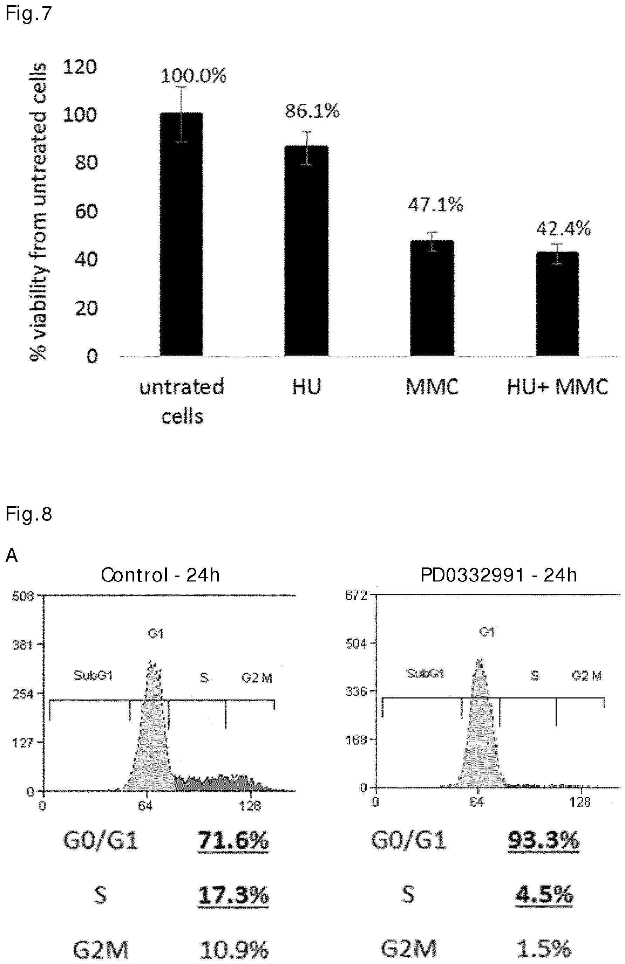

**Figure 7** **shows UMUC-3 cell viability following pretreatment with HU followed by MMC for shorter exposure times.** UMUC-3 cells were pretreated with HU (1mM)/ RPMI media(control) for 2 hours followed by washing, and after 2 hours treatment with MMC/ RPMI media (control) for an additional 2 hours (in a concentration of 20µM, the EC50 value). Cells were washed and cell viability analyzed 48 hours following HU washes. Shown are the results of XTT assays as determined in an ELISA reader at 490nm. The percentage of viable cells was calculated from untreated cells (viability of 100%).

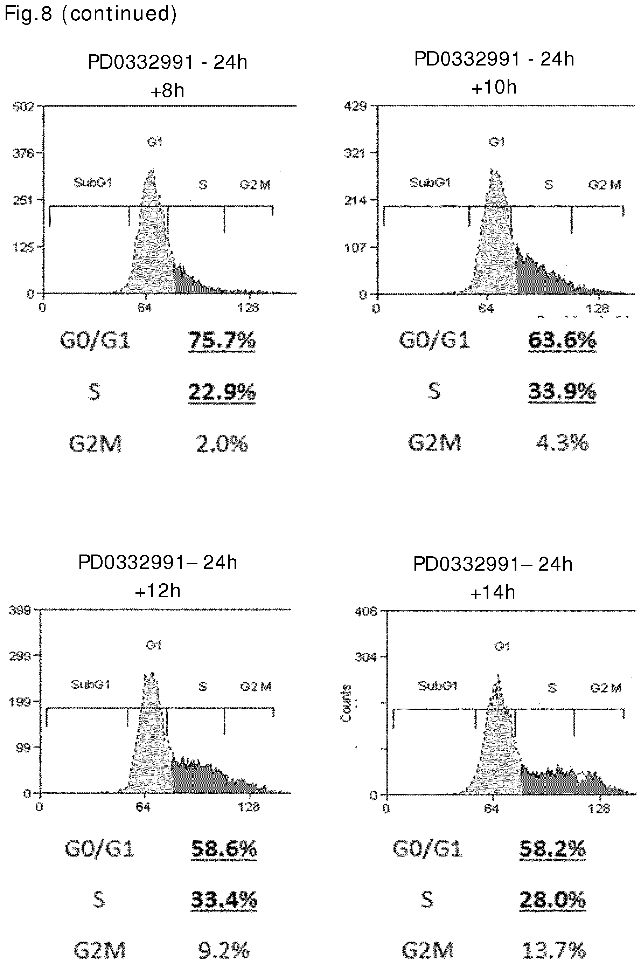

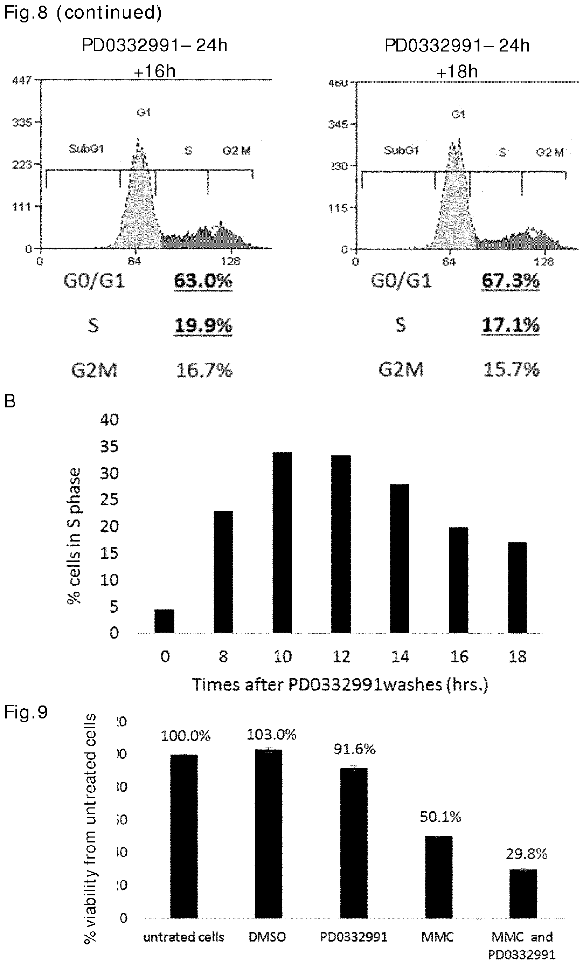

**Figures 8A** **and** **8B** **show cell cycle synchronization at the S phase following PD0332991 exposure.** **Figure 8A****:** UMUC-3 cells were cultured with 0.25µM PD0332991 or with DMSO (control) for 24 hours. Then, PD0332991/DMSO was washed from the cells and replaced by fresh media. Cells were fixed and labeled with PI immediately or between 8 to 18 hours following washes. Cell cycle analysis was done by flow cytometry. The data indicate cells percentage in each cell cycle phase. **Figure 8B****:** A graphical representation showing percentages of UMUC-3 in the S phase following synchronization by PD0332991.
**Figure 9** **shows viability of UMUC-3 bladder cancer cells following combined treatment with PD0332991 followed by MMC.** UMUC-3 cells were pretreated with 0.25µM PD0332991 (or DMSO/ RPMI media [untreated cells]- control) for 24 hours and then washed from the cells with fresh media. Eight hours following washes, cells were treated with 5µM MMC for 17 hours. Cells were washed and cell viability analyzed 48 hours following addition of MMC. Shown are the results of XTT assay as determined in an ELISA reader at 490nm. The percentage of viable cells was calculated from untreated cells (viability of 100%).

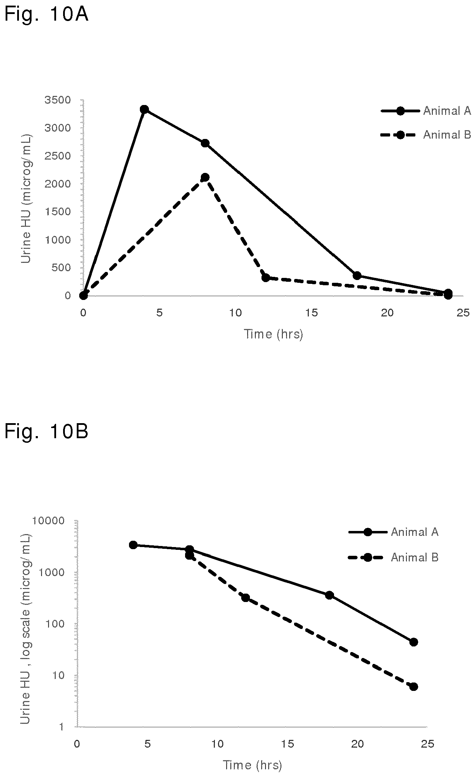

**Figures 10A and 10B** **show the level of HU in pig urine at various time points following intravesical instillation of HU in RTGel (31% Poloxamer 407).** Results for two animals are shown for HU levels (microgram/mL) measured up to 24 hours as raw data (Figure 10A) and log scale (Figure 10B).

### EXAMPLES

### Example 1: Methods

### Cell Culture

The human bladder cancer cell lines, UMUC-3 (high grade human transitional carcinoma bladder tumor cell line) and RT-4 (low grade human transitional cell papilloma bladder tumor cell line), were obtained from the ATCC and grown in RPMI 1640 medium (Gibco) supplemented with 10% v/v fetal calf serum (Gibco) 100U/ml penicillin and 100µg/ml streptomycin (Biological Industries). Cells were grown at 37°C, 5% CO₂ in a humidified atmosphere and harvested using 0.25% w/v trypsin, 0.02% w/v EDTA. Cells were washed in media to inactivate trypsin before reseeding or analysis.

### Drug treatment

Hydroxyurea (HU) was purchased from Sigma Aldrich.
PD0332991 was purchased from Sigma Aldrich.
Mitomycin (MMC) was purchased from TEVA.

Gemcitabine (Gem) and Doxorubicin (Doxo) were purchased from ENCO (Cayman).

Hydroxyurea concentrations were selected based upon concentration ranges known in the art. MMC concentrations were evaluated and an EC₅₀ value was calculated. HU was dosed over a range of MMC/ Doxo/ Gem concentrations and the EC₅₀ value was calculated.

### Cell cycle distribution

A Cyan Flow cytometer was used for determining DNA content and cell cycle distribution of UMUC-3 and RT-4 cells. Cells were seeded in 6 well plates (10⁶ cells/well) and incubated overnight. Cells were then cultured with cell cycle modulator (0.1-15mM HU or 0.25µM PD0332991) or with DMSO (vehicle control) for up to 20 hours (HU) or 24 hours (PD0332991). Then, the HU, PD0332991 (or DMSO) was washed and replaced by fresh media and cells were fixed at different time points following washes. The fixation was done by a fixation buffer that purchased from ENCO (Cayman). Minimum 2 hours after the fixation, cells were washed and labeled with propidium iodide (PI) for 15 min in the dark. Cells were analyzed by flow cytometry (PI staining was detected in FL3, 488nM laser).

### Cell viability

UMUC-3 and RT-4 cells were seeded into 96 well plates (10• /well) and incubated for overnight. Cells were treated with 0-16mM HU/ Control (DMSO or RPMI medium only) for up to 20 hours. Then, 2 hours after HU washes, cells were also treated with sequential administration of MMC/Gem/Doxo for 8 hours. MMC, Gem and Doxo concentrations were evaluated and an EC₅₀ values for the single agents were calculated. Cell viability was detected up to 48 hours from HU washes using the XTT reagent following standard procedures (Biological industries). XTT assay for cytotoxicity was then performed and plates were analyzed in an ELISA reader at wave length of 490nm. In alternative assays, cells were treated with 0.25µM PD0332991 or DMSO for 24 hours prior to washing and then treated with MMC for 17 hours. Viability was measured 48 hours after initiation of MMC treatment.

### Cell cytotoxicity

UMUC-3 and RT-4 cells were seeded into 24 well plates (0.5X10⁶/well) and incubated overnight. Cells were treated with 0.1-15mM HU or control (DMSO or RPMI medium only) for 5-20 hours. Then, cells were washed and MMC/Gem/Doxo (as specified) was added after 2 hours for an additional 8 hours. After 24 hours to 5 days from HU washes, cells were stained with Annexin V FITC and PI for identifying apoptotic cells according to standard protocols. Annexin V-PI kit was purchased from ENCO (Mebcyto). Annexin V and PI activated positive cells were identified by flow cytometer (Cyan) using a single laser emitting excitation light at 488nm.

### Example 2: Cell cycle synchronization by HU

This example shows the synchronization of malignant cells at the S phase of the cell cycle by the addition and withdrawal of HU to cultured cells.

Unless otherwise indicated, methods were as described in Example 1. In order to investigate the influence of HU on cell cycle distribution in bladder cancer cell lines, UMUC-3 and RT-4 cells were treated with 1mM HU (or DMSO for vehicle control) for 20 hours. Cell cycle distribution was observed immediately after HU exposure and 2, 4, 6, 8, and 10 hours following HU withdrawal. As seen in Figures 1A and 1B, treatment with HU resulted in an accumulation of cells at the S phase that arises particularly from 2 to 8 hours after HU washes. UMUC-3 cells demonstrated an approximately 3 fold increase in S phase accumulation (from 18.5% in the control to 57.0% after 4 hours from HU withdrawal), whereas RT-4 cells demonstrated an approximately 6 fold increase (from 8.3% in the control to 48.6% after 4 hours from HU withdrawal). 6 hours and more after HU withdrawal the percentage of cells at the S phase started to decrease and also demonstrated initiation of accumulation of cells at the G2M phase. These results demonstrate that HU can lead to an arrest and synchronization of cells before entering to the S phase, and that following HU washes, cells accumulated at the S phase from about 2 hours and for approximately 8 hours following the wash.

Figures 8A and 8B present a similar experiment with the CDK inhibitor PD0332991. As shown therein, there greatest percentage of UMUC-3 accumulate in S phase 10 and 12 hours after PD0332991 is washed from the cells.

### Example 3: Cytotoxic effect of HU treatment followed by MMC in urinary cancer cell lines

This example shows that sequential, sustained treatment of bladder cancer cells with a cell cycle modulator (HU) followed by a chemotherapeutic agent (MMC) is significantly more effective at killing the cancer cells than treatment with either agent alone.

To investigate the contribution to MMC activity by pretreatment with HU, bladder cancer cell lines were treated with HU followed by MMC. The antiproliferative potential of the combined treatment was evaluated by the XTT cell viability assay. Treatment of UMUC-3 and RT-4 cells only with increased MMC concentrations (between 0 to 160µM) for 8 hours demonstrated a decrease in cell proliferation (Figure 2A, DMSO+MMC and RPMI+MMC). Pretreatment with 1mM HU for 20 hours followed by HU withdrawal and then MMC treatment after 2 hours for 8 hours, showed greater decrease in cell viability (Figure 2A HU+MMC). Furthermore, when comparing viability with approximately 50% of alive cells (EC₅₀) following MMC treatment only to cells that were pretreated also with HU the differences in viability between MMC alone and HU + MMC were determined to be statistically significant (Figure 2B, averages of at least 4 independent experiment for each cell line).

In order to investigate the effect of the combined treatment (HU followed by MMC) on cell apoptosis, UMUC-3 and RT-4 cells were pretreated with 1mM HU for 20 hours followed by HU washes and addition of MMC after 2 hours for 8 hours. 48 hours after HU withdrawal, cell apoptosis was evaluated by Annexin V FITC and PI staining. As shown in Figure 2C, UMUC-3 and RT-4 cells that were treated with HU+MMC demonstrated statistically significant higher percentages of apoptotic cells compared to cells that were treated with HU or MMC alone (in UMUC-3 cells: 51.2% - HU+MMC vs. 21.4%- MMC and 8.1%-HU. In RT-4 cells: 24.4%-HU+MMC vs. 13.4%-MMC and 4.8%- HU). Thus, the combined treatment of HU followed by MMC compared to each drug alone demonstrated a significantly higher cytotoxicity, as shown in decreased cell viability and increased cell apoptosis. As shown in Figure 9, a greater than additive killing effect can also be seen with sequential exposure of UMUC-3 cells to the CDK inhibitor PD0332991, followed by MMC.

### Example 4: Simultaneous and Reverse Sequential Treatment with HU and MMC

The previous examples demonstrate a synergistic effect of the sequential treatment with HU followed by MMC. Figures 3A-B compare the percentages of cell viability in response to sequential treatment of MMC followed by HU (*i.e.* the opposite of the sequence described as providing treatment synergy). UMUC-3 cells were treated with MMC for 8 hours at the noted concentrations, followed by a 2 hour wash and then 1mM HU for 20 hours. Cell viability was measured by XTT assay. As shown in Figures 3A and 3B, when the sequence of treatments was reversed (MMC provided first, followed by HU), there was no significant difference in the effect on cell viability between the sequential treatment and the treatment with MMC alone (50.1% - MMC vs. 47.6%- MMC followed by HU). These results clearly demonstrate the significance of the sequence of treatment provided.

The difference in cell viability (%) between sequential treatment of HU followed by MMC and that of simultaneous HU and MMC treatment is shown in Figure 3C. In the "reverse sequential" experiments, MMC was applied to UMUC-3 cells for 8 hours under standard culture conditions. 2 hours following washes, the cells were treated with HU for 20 hours, after which cell viability was determined by XTT assay as described, and in comparison, to untreated cells. In the "simultaneous" experiments, cells were treated with identical HU and MMC concentrations for 20 hours, and cell viability determined. To control for unavoidable differences in MMC exposure times (8 hours for the sequential treatment vs. 20 hours for the simultaneous treatment), we deducted the viability percentages of the combined treatment from the viability percentages following MMC treatment alone (for each of the relevant MMC exposure time). This enabled us to eliminate the influence of the differences in MMC exposure time and highlighted the added value of the combined treatment with HU (HU exposure was kept for the same time duration). The results are shown in the bar graph of Figure 3C. As shown therein, the effect on the difference in cell viability (viability of MMC alone minus that of the combined treatment), is significantly greater in the sequential treatment than in the simultaneous treatment, demonstrating that the sequential treatment (HU followed by MMC) provides a greater effect on viability and hence, greater reduction in the number of viable cells compared to the simultaneous treatments.

This example demonstrates that providing the recited treatments simultaneously or in a reverse sequence to that described above does not provide the described benefit.

### Example 5: Treatment with various HU concentrations for different periods of times followed by MMC

To assess the effect of HU exposure time on the outcome of HU+MMC combined treatment effectively, UMUC-3 and RT-4 cells were exposed first to HU for 5, 12 or 20 hours, and the cell cycle distribution was determined. Following each period, HU was washed and cells were fixed every 2 hours in order to monitor accumulation of cells at the S phase. As shown in Figure 4A, the percentage of cells that accumulated at the S phase was usually lower following 5 hours of treatment compared to 12 or 20 hours.

To compare the efficiency of the combined treatment (HU+MMC) after 12 or 20 hours of HU pre-exposure, cell viability and cell apoptosis were analyzed. UMUC-3 and RT-4 cells were treated with 1mM HU for 12 hours or for 20 hours followed by MMC. Figure 4B presents cell viability data from averages of five (UMUC-3) or four (RT-4) independent experiments. In both UMUC-3 and RT-4 cells, decreased cell viability following the combined treatment was statistically higher following 20 hours compared to 12 hours HU exposure (UMUC-3: 30.8%/ 23.1% for 12/20 hours respectively, p<0.05,• t-test. RT-4: 30.4%/20.5% for 12/20 hours respectively, p<0.05• t-test). In comparing cytotoxicity of cells by annexin-PI staining to detect apoptosis, slightly greater apoptosis percentage following 20 hours HU exposure compared to 12 hours HU exposure (representative experiments in Figure 4C [statistical analysis of four independent experiments for UMUC-3 cells, p=0.08,• t-test. Statistical analysis of three independent experiments for RT-4, p=0.1,• t-test]).

To assess the impact of pretreatment with different HU concentrations on S-phase synchronization, UMUC-3 and RT-4 cells were first treated with various HU concentrations between 0.1 to 15mM for 20 hours. After HU withdrawal, S phase accumulation was detected every 2 hours. Figure 5A and 5B show the S phase percentages graphs following HU treatments on UMUC-3 cells (Figure 5A: 0.1-4mM, Figure 5B: 7-15mM). The results demonstrate that in a concentration range of 0.5 to 4mM, S phase accumulation was greater than 50%, 6 hours after the HU wash. From 10mM and further on, the increase was much lower. Similar observations were made in RT-4 cells. HU concentrations lower than 0.1mM were also investigated, but no significant influence on cell cycle distribution was observed.

To investigate the influence of HU concentration on the sequential combination treatment efficacy, UMUC-3 cells were treated with 0-16mM HU followed by a fixed MMC concentration of 5 or 20µM MMC, and cell viability was assessed. From 1 to 8mM HU, a similar percentage viability was detected (Figure 5C). In addition, in most of the assessed HU concentrations, the combined treatment with MMC (5µM or 20µM) demonstrated significantly decreased cell viability in comparison to cells that were treated with only one agent. To understand the effects on cytotoxicity, the annexin-PI assay was performed following the combined treatment of various HU concentrations followed by MMC. As shown in Figure 5D, UMUC-3 cells were treated with 0.1-15mM HU for 12 hours or 20 hours followed by 8 hours of 20µM MMC, after which the amount of cell apoptosis was determined. As demonstrated therein, apoptosis levels following HU+MMC treatment increased in direct proportion to the rising HU concentrations, especially following 20 hours of HU treatment. When comparing the effect following 12 hours vs. 20 hours HU treatment, the apoptosis percentages were significantly higher following 20 hours of HU treatment, and particularly at a concentration of 2mM and higher. For example, for 4mM HU concentration in combination with MMC, 22.9% apoptosis was observed after 12 hours vs. 41.6% after 20 hours. Similar results were detected on RT-4 cells.

### Example 6: Cytotoxic effect of HU followed by other chemotherapeutic drugs in bladder cancer cell lines

The previous examples demonstrated that that sequential, sustained treatment of bladder cancer cells with a cell cycle modulator (HU) and a chemotherapeutic agent (MMC) is significantly more effective at killing the cancer cells than treatment with either agent alone. This example demonstrates the efficacy of sequential combinations using HU as a cell cycle modulator to enhance the efficacy of other, non-MMC chemotherapeutics, particularly Gemcitabine (Gem) and Doxorubicin (Doxo).

To investigate the efficacy of combinations of HU+ Gem, and also combination of HU followed by Doxo on bladder cancer cells, UMUC-3 and RT-4 cells were pretreated with HU for 20 hours followed by Gem or Doxo, and cell viability and apoptosis assays were performed as above. Both UMUC-3 and RT-4 cells were pretreated with 1mM HU followed by exposure to increasing concentrations of Gem and Doxo in the range of 0-2000nM (0-2µM) and 0-250µM respectively. Figure 6A shows a comparison of percent viability between untreated, singly treated, or combined treated. The results demonstrate large, and even synergistic cell viability decreases following the combined treatment when compared to controls and to treatments with only one agent in both UMUC-3 and RT-4 cells. For example, 21.8% vs. 50.9% and 71.3% viability for HU+ Gem vs. Gem and HU respectively in UMUC-3 cells. Furthermore, as shown in Figure 6B, the combinations of HU+ Gem / HU+ Doxo demonstrated synergistically higher apoptosis percentages in comparison to each drug alone in both UMUC-3 and RT-4 cells (e.g. 53.2% vs. 22.2% and 5.9% late apoptosis for HU+ Gem vs. Gem and HU respectively in RT-4 cells). Thus, we conclude that as with MMC, pretreatment with HU can improve the cytotoxic effect of other chemotherapy drugs such as Gem and Doxo.

### Example 7: Shortened Sequential Treatment with HU and MMC with a Washout Period

In the previous examples, sustained exposure to HU and MMC was used to mimic the effects of administering the active agents to a subject within the context of a hydrogel composition that can remain in contact with targeted cells for an extended period of time (at least 6-24 hours). This example presents the effects of a greatly shortened exposure to HU followed by MMC, as might be experienced by a patient provided with a non-hydrogel based treatment.

The methods used were as described in the other examples. Briefly, UMUC-3 cells were cultured in 96 well microculture plates. Cells were pretreated with 1mM HU (or DMSO- vehicle control) for 2 hours. Cells were then washed and 2 hours after the wash the cells were treated with 20 µM of MMC for 2 hours. MMC was then replaced by fresh medium. Cell viability was analyzed 48 hours following the addition of MMC by the XTT assay in an ELISA reader at wave length of 490nm. The percentage of viable cells presented in the figures and table was calculated from untreated cells with viability of 100%.

Treatment with HU or MMC caused increased cell cytotoxicity (cell viability followed addition of HU or MMC was 86.1% and 47.1%, respectively). Sequential addition of the compounds increased cell death considerably mildly (cell viability followed sequential addition of HU and MMC was 42.4%). Results are presented in Figure 7 and Table 1.

**Table 1: Cytotoxicity of Short-Term, Sequential HU and MMC Exposure with Intervening Delay**

| **Drug treatment** | **Concentration of MMC** | **Concentration of HU** | **% Viability** |
|---|---|---|---|
| MMC | 20µM | NA | 47.1% |
| HU | NA | 1mM | 86.1% |
| HU (2 hours) followed by 2 hours wash and MMC (2 hours) | 20µM | 1mM | 42.4% |

| | | | |
|---|---|---|---|
| NA- not applicable | | | |

### Example 8: In vivo intravesical instillation of HU in RTGel

This example shows the level of hydroxyurea (HU) in pig urine at various time points following *in vivo* intravesical instillation of HU in RTGel thermoreversible hydrogel.

### Materials

HU powder was manufactured by Euticals. The HU-RTGel formulation was prepared by UroGen Chemistry Lab and contained 31% Poloxamer 407 and 22.2 mg/mL of HU.

### Study description

Two female domestic pigs aged 4-5 months were used. Following anesthesia, a baseline urine sample was obtained by ultrasound guided cystocentesis. A Foley catheter (18Fr) was then inserted into the urinary bladder the pig was subjected to a single intravesical instillation of 90mL HU-RTGel admixture (total dose of 2gr HU), following urine drainage. The catheter was removed 45 minutes following instillation while the animal remained non-mobile for ∼ 60 minutes. Then, the animal was allowed to wake up and was moved to a study-designated cage where urine was collected for 24 hours following instillation.

### PK analysis

An LC-MS/MS method (Analyst Research Laboratories) was used for the quantitative determination of HU in pig urine. PK analysis was based on urine samples from each animal, using urine volume for each interval time.

### Results

Intravesical instillation of HU-RTGel resulted in measurable HU in the urine for 24 hours (last sample collected, as shown in Figures 10A and 10B). Highest HU urine concentrations were obtained between 4 to 8 hours following instillation and were higher than 2000µg/mL. Furthermore, HU levels in urine remained high for at least 12 hours in both animals following instillation, higher than 300µg/mL. In the *in* vitro results shown in Figures 1A and 1B, a HU concentration of 76µg/mL (1mM) was shown to induce cell cycle synchronization at the S stage.

### Conclusions

Intravesical instillation of HU-RTGel (90mL of 22mg/mL HU, 31% Poloxamer 407) in 2 domestic pigs resulted in urine levels of HU that are above the concentration found *in vitro* to induce cell cycle arrest and synchronization at the S stage for at least 12 hours.

In view of the many possible embodiments to which the principles of the disclosed invention may be applied, it should be recognized that the illustrated embodiments are only preferred examples of the invention and should not be taken as limiting the scope of the invention. Rather, the scope of the invention is defined by the following claims. We therefore claim as our invention all that comes within the scope and spirit of these claims.

## Claims

1. A cell cycle modulator capable of arresting the cell cycle in the S and/or G1 phase for the treatment of cancer of a body cavity, particularly wherein the cancer of the body cavity is a urinary tract cancer, particularly bladder cancer or upper tract urothelial carcinoma (UTUC),
wherein the cell cycle modulator is administered comprised in a first composition comprising a first sustained release composition, particularly a thermoreversible hydrogel composition, administered locally to said body cavity,
followed by administration of a second composition comprising at least one anti-cancer agent to said body cavity.

2. The cell cycle modulator for the treatment of cancer of a body cavity according to claim 1, wherein the second composition comprises a second sustained release composition, particularly a thermoreversible hydrogel composition.

3. The cell cycle modulator capable for the treatment of cancer of a body cavity according to claim 1 or 2, wherein the second composition is to be administered to the body cavity 2-196 hours, particularly 3 to 120, 8 to 36, 12 to 24, hours, more particularly 4 to 48 hours, after the first composition is administered to the body cavity.

4. The cell cycle modulator capable for the treatment of cancer of a body cavity according to any one of the preceding claims, wherein the concentration of the cell cycle modulator in the first composition ranges from 0.5mmol/L to 1.5 mol/L, particularly from 0.5mmol/L to 1.5 mol/L, particularly from 2 mmol/L to 250 mmol/L, more particularly from 10 mmol/L to 100 mmol/L.

5. The cell cycle modulator capable for the treatment of cancer of a body cavity according to any one of the preceding claims, wherein the cell cycle modulator is selected from the group consisting of: hydroxyurea, alternol, resveratrol, fluorodeoxyuridine, thymidine dinucleotide pTpT and Zidovudine, PD-0332991, LEE011, CINK4, Flavopiridol, p276-00 and P1446A-05.

6. The cell cycle modulator capable for the treatment of cancer of a body cavity according to any one of the preceding claims, wherein the anti-cancer agent is selected from the group consisting of: Mitomycin C, Apaziquone, Gemcitabine, Cisplatin, 5-FU (5- fluorouracil) Doxorubicin, Valrubicin, Epirubicin, Taxol, Pirarubicin and Methotrexate.

7. The cell cycle modulator capable for the treatment of cancer of a body cavity according to any one of the preceding claims 2 to 6, wherein said first sustained release composition is the same as the second sustained release composition.

8. A combination medicament comprising
- a first composition comprising a first sustained release composition component, particularly a first thermoreversible hydrogel component, and a cell cycle modulator capable of arresting the cell cycle in the S and/ or G1 phase, and
- a second composition comprising a second sustained release composition component, particularly a second thermoreversible hydrogel component, comprising at least one chemotherapeutic agent.

9. The combination medicament according to claim 8, wherein the concentration of the cell cycle modulator in the first composition ranges from 0.5mmol/L to 1.5 mol/L, particularly from 0.5mmol/L to 1.5 mol/L, particularly from 2 mmol/L to 250 mmol/L, more particularly from 10 mmol/L to 100 mmol/L.

10. The combination medicament according to claim 8 or 9, wherein the cell cycle modulator is selected from the group consisting of: hydroxyurea, alternol, resveratrol, fluorodeoxyuridine, thymidine dinucleotide pTpT and Zidovudine, PD-0332991, LEE011, CINK4, Flavopiridol p276-00 and P1446A-05.

11. The combination medicament according to any one of claims 8 to 10, wherein the chemotherapeutic agent is selected from the group consisting of: Mitomycin C, Apaziquone, Gemcitabine, Cisplatin, 5-FU (5- fluorouracil) Doxorubicin, Valrubicin, Epirubicin, Paclitaxel, Pirarubicin, Methotrexate and analogs thereof.

12. The combination medicament according to any one of claims 8 to 11, wherein the first and/or second sustained release composition component comprises or is essentially constituted of:
- 20% to 35% (w/w) PEO/PPO block copolymer and
- optionally at least one mucoadhesive agent, particularly a mucoadhesive agent selected from hydroxypropylmethylcellulose (HPMC) and Polyvinylpyrrolidone (PVP).

13. The combination medicament according to any one of claims 8 to 12 for the treatment of cancer of a body cavity, particularly for the treatment of urinary tract cancer, more particularly for the treatment of bladder cancer and upper tract urothelial carcinoma, by local administration to said body cavity, particularly by intravesical instillation of the first and the second composition.

14. The combination medicament for the treatment of cancer of a body cavity according to claim 13, wherein
a. said first composition is administered for at least 2 hours, particularly for at least 12 hours; and / or
b. the second composition is administered after the first composition has been washed from the body cavity; and / or
c. the second composition is to be administered to the body cavity 2-168 hours after the first composition is administered to the body cavity.

15. A packing unit comprising
a first composition comprising a first sustained release composition component, particularly a first thermoreversible hydrogel component, and a cell cycle modulator capable of arresting the cell cycle in the S phase and/or G1 phase, particularly a cell cycle modulator selected from hydroxyurea, alternol, resveratrol, fluorodeoxyuridine, thymidine dinucleotide pTpT, Zidovudine, PD-0332991, LEE011, CINK4, Flavopiridol p276-00 and P1446A-05;
a second composition comprising a second sustained release composition component, particularly a second thermoreversible hydrogel component, and at least one chemotherapeutic agent, particularly a chemotherapeutic agent selected from Mitomycin C, Apaziquone, Gemcitabine, Cisplatin, 5-FU (5- fluorouracil) Doxorubicin, Valrubicin, Epirubicin, Pirarubicin and Methotrexate.
